(19) Europäisches Patentamt
European Patent Office
Office européen des brevets

(11) EP 3 253 301 B1

(12) **EUROPEAN PATENT SPECIFICATION**

(45) Date of publication and mention of the grant of the patent:
**05.06.2019 Bulletin 2019/23**

(21) Application number: **16706477.3**

(22) Date of filing: **05.02.2016**

(51) Int Cl.:
***A61B 17/12*** *(2006.01)* ***A61B 17/00*** *(2006.01)*

(86) International application number:
**PCT/US2016/016814**

(87) International publication number:
**WO 2016/127078 (11.08.2016 Gazette 2016/32)**

(54) **OCCLUSION DEVICES**

OKKLUSIONSVORRICHTUNGEN

DISPOSITIFS D'OCCLUSION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.02.2015 US 201562113111 P**

(43) Date of publication of application:
**13.12.2017 Bulletin 2017/50**

(73) Proprietor: **Boston Scientific Scimed, Inc.**
**Maple Grove, MN 55311 (US)**

(72) Inventors:
- **SQUIRE, Robert, N.**
  **Maple Grove, MN 55369 (US)**
- **LINDQUIST, Jeffrey, S.**
  **Maple Grove, MN 55311 (US)**
- **HARRISON, Kent, D.**
  **Maple Grove, MN 55311 (US)**
- **HORN, Daniel, J.**
  **Shoreview, MN 55126 (US)**
- **KANGAS, Steven, L.**
  **Woodbury, MN 55125 (US)**

(74) Representative: **Vossius & Partner**
**Patentanwälte Rechtsanwälte mbB**
**Siebertstrasse 3**
**81675 München (DE)**

(56) References cited:
**WO-A1-2013/068466**
**US-A1- 2002 082 638**
**US-A1- 2003 220 666**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 253 301 B1

## Description

TECHNICAL FIELD

This disclosure relates to endovascular devices, and more particularly, to vaso-occlusive devices for the occlusion of body lumens and cavities.

BACKGROUND

In many clinical situations, blood vessels are occluded for a variety of purposes, such as to control bleeding, to prevent blood supply to tumors, and to block blood flow within an aneurysm. Vaso-occlusive devices have been used in the treatment of aneurysms. Vaso-occlusive devices are surgical implants placed within blood vessels or vascular cavities, typically by the use of a catheter, to form a thrombus and occlude the site. For instance, an aneurysm may be treated by introduction of a vaso-occlusive device through the neck of the aneurysm. The thrombogenic properties of the vaso-occlusive device cause a mass to form in the aneurysm and alleviate the potential for growth of the aneurysm and its subsequent rupture. Other diseases, such as tumors, may also be treated by occluding the blood flow to a target area. Document US2003220666 discloses features falling under the preamble of claim 1.

SUMMARY

The invention is defined in claim 1. Further embodiments of the invention are defined in the dependent claims. This disclosure relates to endovascular devices, and more particularly, to vaso-occlusive devices for the occlusion of body lumens and cavities. In one illustrative embodiment, a medical device can include a catheter shaft extending from a proximal end to a distal end. The catheter shaft may further include a plurality of lumens extending through at least a portion of the catheter shaft. A balloon member is disposed proximate the distal end of the catheter shaft. The catheter shaft further comprises a frangible portion disposed proximate the distal end of the catheter shaft for detaching the balloon member from the catheter shaft. At last two of the plurality of lumens merge into a single lumen.

Alternatively, or additionally, the catheter shaft may have a first wall thickness proximal of the frangible portion, the frangible portion may have a second wall thickness, and the second wall thickness may be less than the first wall thickness.

Alternatively, or additionally, the frangible portion may comprise one or more perforations through the catheter shaft.

Alternatively, or additionally, the frangible portion may comprise one or more discontinuous recesses in an outer wall of the catheter shaft.

Alternatively, or additionally, the frangible portion may be disposed proximal of the balloon member.

Alternatively, or additionally, the frangible portion may be disposed on the balloon member.

Alternatively, or additionally, the catheter shaft may further comprise a mixing region disposed proximate the distal end of the shaft.

Alternatively, or additionally, the mixing region may comprise one or more barriers extending part way into one of the plurality of lumens.

Alternatively, or additionally, the mixing region may comprise a static helical mixer.

Alternatively, or additionally, the medical device may further comprise a biologically safe adhesive disposed on an outside of the balloon member.

Alternatively, or additionally, the balloon member may be integral with the catheter shaft.

Alternatively, or additionally, the balloon member may be a separate component from the catheter shaft.

Alternatively, or additionally, the balloon member may be connected to the catheter shaft with an adhesive that is soluble in an aqueous environment of blood.

Alternatively, or additionally, the balloon member may be compliant and configured to stretch to assume a shape of a vessel in which the balloon member is contained.

Alternatively, or additionally, one or more of the plurality of lumens may be a guidewire lumen.

Alternatively, or additionally, none of the plurality of lumens is a guidewire lumen.

In another illustrative embodiment, a medical device may comprise a catheter shaft having a distal end and a proximal end and including a plurality of lumens extending through at least a portion of the catheter shaft. In some embodiments, the medical device may further include a balloon member disposed proximate the distal end of the catheter shaft, and the catheter shaft may comprise a frangible portion disposed proximate the distal end of the catheter shaft for detaching the balloon member from the catheter shaft.

Alternatively, or additionally, the catheter shaft may have a first wall thickness proximal of the frangible portion, the frangible portion may have a second wall thickness, and the second wall thickness may be less than the first wall

thickness.

Alternatively, or additionally, the frangible portion may comprise one or more perforations through the catheter shaft.

Alternatively, or additionally, the frangible portion may comprise one or more discontinuous recesses in an outer wall of the catheter shaft.

Alternatively, or additionally, the frangible portion may be disposed proximal of the balloon member.

Alternatively, or additionally, the frangible portion may be disposed on the balloon member.

Alternatively, or additionally, the catheter shaft may further comprise a mixing region disposed proximate the distal end of the catheter shaft.

Alternatively, or additionally, the mixing region may comprise a static helical mixer.

Alternatively, or additionally, the balloon member may be a separate component from the catheter shaft and adhesively or thermally connected to the catheter shaft.

Alternatively, or additionally, the adhesive may be soluble in an aqueous environment of blood.

Alternatively, or additionally, the balloon member may be integral with the catheter shaft.

Alternatively, or additionally, one of the plurality of lumens may be a guidewire lumen.

Alternatively, or additionally, none of the plurality of lumens is a guidewire lumen.

Concerning the invention a medical device comprises a catheter shaft having a distal end and a proximal end and including a plurality of lumens extending through at least a portion of the catheter shaft. At least two of the plurality of lumens merge into a single lumen. The medical device further includes a balloon member disposed at the distal end of the catheter shaft, wherein the single lumen opens into the balloon member. The catheter shaft further includes a frangible portion disposed near the distal end of the catheter shaft for detaching the balloon member from the catheter shaft.

Alternatively, or additionally, the catheter shaft may have a first wall thickness proximal of the frangible portion, the frangible portion may have a second wall thickness, and the second wall thickness may be less than the first wall thickness.

Alternatively, or additionally, the frangible portion may comprise one or more perforations through the catheter shaft.

Alternatively, or additionally, the frangible portion may comprise one or more discontinuous recesses in an outer wall of the catheter shaft.

Alternatively, or additionally, the catheter shaft may further comprise a mixing region disposed proximate the distal end of the catheter shaft.

Alternatively, or additionally, the mixing region may comprise a static helical mixer.

In yet another illustrative embodiment, a method of forming a medical device comprises forming a catheter shaft having a proximal end and a distal end and including a plurality of lumens extending through at least a portion of the catheter shaft. The method may further comprise forming a balloon member on the catheter shaft proximal the distal end of the catheter shaft. In some embodiments, the method may further include weakening a distal portion of the catheter shaft to create a frangible region.

Alternatively, or additionally, the balloon member may be formed separately from the catheter shaft and attached to the catheter shaft.

The above summary of the present disclosure is not intended to describe each embodiment or every implementation of the present disclosure. Advantages and attainments, together with a more complete understanding of the disclosure, will become apparent and appreciated by referring to the following detailed description and claims taken in conjunction with the accompanying drawings.

## BRIEF DESCRIPTION OF THE DRAWINGS

The disclosure may be more completely understood in consideration of the following detailed description of various embodiments in connection with the accompanying drawings, in which:

Figure 1 is a side plan view of a catheter in accordance with various embodiments of the present disclosure:

Figures 2A-2C are example cross-sectional views of the catheter shown in Figure 1 as viewed along line A-A, in accordance with various embodiments of the present disclosure;

Figures 3A and 3B are example views of the distal region of the catheter shown in Figure 1 with the balloon member in an un-inflated state, in accordance with various embodiments of the present disclosure;

Figure 3C is an example view of the distal region of the catheter shown in Figure 1 including a guidewire port, in accordance with various embodiments of the present disclosure;

Figure 3D is an example cross-sectional view of a frangible region of the catheter shown in Figure 3C, as viewed along line E-E of Figure 3C;

Figure 3E is an example view of the distal region of the catheter shown in Figure 1 including a guidewire port, in accordance with various embodiments of the present disclosure;

Figure 3F is an example cross-sectional view of a frangible region of the catheter shown in Figure 3E, as viewed along line F-F of Figure 3E;

Figures 4A and 4B are example views of the distal region of the catheter shown in Figure 1 with the balloon member in an inflated state;

Figure 5A is an example cross-sectional view of the distal region of the catheter shown in Figure 1 including a frangible region, in accordance with various embodiments of the present disclosure;

Figures 5B and 5C are example cross-sectional views of the distal region of the catheter shown in Figure 5A as viewed along line B-B of Figure 5A;

Figure 6 is a cross-sectional view of the distal region of the catheter shown in Figure 1 including a frangible region, in accordance with various embodiments of the present disclosure;

Figure 7 is an example plan view of the distal region of the catheter shown in Figure 1 including a frangible region, in accordance with various embodiments of the present disclosure;

Figure 8 is an example plan view of the distal region of the catheter shown in Figure 1 including a frangible region, in accordance with various embodiments of the present disclosure;

Figure 9A is an example plan view of the distal region of the catheter shown in Figure 1 including a frangible region, in accordance with various embodiments of the present disclosure;

Figure 9B is an example cross-sectional view of the catheter shown in Figure 9A as viewed along line C-C, in accordance with various embodiments of the present disclosure;

Figure 10A is an example plan view of the distal region of the catheter shown in Figure 1 including a frangible region, in accordance with various embodiments of the present disclosure;

Figure 10B is an example cross-sectional view of the catheter shown in Figure 10A, in accordance with various embodiments of the present disclosure;

Figure 11 is an example cross-sectional view of the distal region of the catheter shown in Figure 1 including a detachable balloon member, in accordance with various embodiments of the present disclosure;

Figure 12A is an example cross-sectional view of the distal region of the catheter shown in Figure 1 with an integral balloon member in a deflated state, in accordance with various embodiments of the present disclosure;

Figure 12B is an example cross-sectional view of the distal region of the catheter shown in Figure 1 with an integral balloon member in an inflated state, in accordance with various embodiments of the present disclosure;

Figures 13A-13D are example views of the catheter of Figure 1 in relation to a body cavity, in accordance with various embodiments of the present disclosure;

Figures 14A-C are additional example views of the catheter of Figure 1 in relation to a collateral vessel, in accordance with various embodiments of the present disclosure;

Figure 15 is an example cross-sectional view of the distal region of the catheter shown in Figure 1 including a mixing region, in accordance with various embodiments of the present disclosure;

Figure 16 is an example cross-sectional view of the distal region of the catheter shown in Figure 1 including a mixing region, in accordance with various embodiments of the present disclosure;

Figure 17 is an example cross-sectional view of the distal region of the catheter shown in Figure 1 including a mixing region, in accordance with various embodiments of the present disclosure; and

Figure 18 is a flow diagram of an illustrative method for forming a medical device, for example catheter 10 as depicted with respect to Figure 1.

While the disclosure is amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the scope of the disclosure.

DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (i.e., having the same function or result). In many instances, the term "about" may be indicative as including numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

Although some suitable dimensions, ranges and/or values pertaining to various components, features and/or specifications are disclosed, one of skill in the art, incited by the present disclosure, would understand desired dimensions, ranges and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the disclosure. The illustrative embodiments depicted are intended to be only exemplary. Selected features of any illustrative embodiments may be incorporated into any other described embodiments unless clearly stated to the contrary.

Figure 1 shows an exemplary catheter 10 in accordance with various embodiments of the present disclosure. In some cases, catheter 10 may be a guide or diagnostic catheter, and may have a length and an outside diameter appropriate for its desired use, for example, to enable intravascular insertion and navigation. For example, when catheter 10 is adapted as a guide catheter, catheter 10 may have a length of about 20-250 cm and an outside diameter of approximately 1-10 French, depending upon the desired application. In some cases, catheter 10 may be a microcatheter that is adapted and/or configured for use within small anatomies of the patient. For example, catheter 10 may be used to navigate to targets sites located in tortuous and narrow vessels such as, for example, to sites within the neurovascular system, certain sites within the coronary vascular system, or to sites within the peripheral vascular system such as superficial femoral, popliteal, or renal arteries. In some cases, the target site is a neurovascular site and may be located within a patient’s brain, which is accessible only via a tortuous vascular path. However, it is contemplated that the catheter may be used in other target sites within the anatomy of a patient. An exemplary catheter that may be utilized in accordance with the various embodiments as described herein is shown and described in U.S. Patent 8,182,465.

As shown in Figure 1, catheter 10 can include elongate catheter shaft 12. Elongate shaft 12 may generally extend from proximal portion 16 and proximal end 18 in distal direction D toward distal portion 20. Although depicted as having a generally circular cross-sectional shape, it will be understood that elongate shaft 12 can have other cross-sectional shapes or combinations of shapes without departing from the scope of the disclosure. For example, the cross-sectional shape of the generally tubular elongate shaft 12 may be oval, rectangular, square, triangular, polygonal, and the like, or any other suitable shape, depending upon the desired characteristics.

In some cases, manifold 14 may be connected to proximal end 18 of elongate shaft 12. The manifold may include hub 17 and/or other structures to facilitate connection to other medical devices (e.g., syringe, stopcocks, Y-adapter, etc.) and to provide access to one or more lumens defined within elongate shaft 12. In some cases, hub 17 may include ports 6, 7, and 8, which provide individual access to one or more lumens extending through at least a portion of catheter 10. Some example lumens that may extend through catheter 10 may include at least one guidewire lumen and one or more inflation lumens. The lumens that do extend through catheter 10 may terminate at or near distal portion 20 of elongate shaft 12, as will be described with respect to other figures. However, in other cases, hub 17 may have a single port, two ports, or any other number of ports. Manifold 14 may also include a strain relief portion adjacent proximal end 18 of elongate shaft 12.

Distal portion 20 of elongate shaft 12 may include balloon member 25, shown in Figure 1 in a deflated state. In accordance with techniques described herein in greater detail, balloon member 25 may be detached from catheter 12 and act as an occlusive body. To facilitate detachment of balloon member 25 from elongate shaft 12, elongate shaft 12 may include frangible region 24 disposed on distal portion 20 of elongate shaft 12. Figure 1 depicts frangible region 24 including perforations 27, however in other embodiments frangible region 24 may include additional or different features, as will be described in more detail below. In some cases, elongate shaft 12 may include additional devices or structures such as inflation or anchoring members, sensors, optical elements, ablation devices or the like, depending upon the desired function and characteristics of catheter 10.

Figures 2A-2C are all example cross-sections of elongate shaft 12 viewed along line A-A as depicted in Figure 1. As described previously, elongate shaft 12 may have multiple lumens extending through at least a portion of elongate shaft 12, for example lumens 31, 33, and 35. In some embodiments, lumen 33 may be a guidewire lumen and lumens 31, 35 may be inflation lumens. Lumens 31, 33, and 35 may all be separate lumens along at least a portion of elongate shaft 12. Figure 2A depicts an example cross section of elongate shaft 12 where elongate shaft 12 has been made from a solid cylinder of material with lumens 31, 33, and 35 set into the material and separate from outer wall 13 of elongate shaft 12. In these embodiments, elongate shaft 12 may be formed, for example, by extrusion. Figure 2B depicts an example cross section of elongate shaft 12 where lumens 31, 33, and 35 are coextensive with at least a portion of wall 15 of elongate shaft 12 and separated by barrier 32 extending through elongate shaft 12. Figure 2C depicts elongate shaft 12 where lumens 31, 33, and 35 are created by separate tubes 34, 36, and 38 all residing within elongate shaft 12 lumen 39 of elongate shaft 12.

Figure 3A depicts a close-up of distal portion 20 of elongate shaft 12, according to some embodiments of the

present disclosure. Figure 3A depicts lumens 31, 33, and 35 extending through distal portion 20 of elongate shaft 12. Figure 3A also depicts balloon member 25 in cross-section disposed around distal portion 20. In some embodiments, as depicted in Figure 3A, lumens 31, 33, and 35 may extend all the way to distal end 22 of elongate shaft 12. In the embodiment of Figure 3A, lumens 31 and 35 may be inflation lumens and may have inflation ports 93 and 95 which connect inflation lumens 31 and 35 to the interior of balloon member 25. Accordingly, when inflation media is pushed through inflation lumens 31 and 35, the inflation media may enter balloon 25 through inflation ports 93, 95 and inflate balloon member 25. In such embodiments, the distal ends of each inflation lumen 31, 35 may be sealed. In some of these embodiments, the distal end of guidewire lumen 33 may extend through balloon member 25 to a distal guidewire opening, or guidewire lumen 33 may terminate at a different location. For example, guidewire lumen 33 may terminate with a distal guidewire opening proximal of balloon 25 such that a guidewire may pass along an exterior of balloon 25. In other instances, elongate shaft 12 may not include a guidewire lumen 33. In some such instances, elongate shaft 12 may or may not include one or more additional lumens, such as a lumen for receiving a stiffening member or stylet therein.

Figure 3B depicts another close-up of distal portion 20 of elongate shaft 12, in accordance with some embodiments. In the embodiment of Figure 3B, inflation lumens 31, 35 may terminate before distal end 22. For instance, inflation lumens 31, 35 may merge to form a single lumen 37. Inflation ports 93, 95 may then connect lumen 37 to the interior of balloon member 25. Although Figure 3B depicts inflation lumens 31, 35 merging proximal of frangible region 24, in other examples, inflation lumens 31, 35 may merge distal of frangible region 24 (with proximal and distal directions identified by arrows P and D). For instance, inflation lumens 31, 35 may merge distal of frangible region 24, but proximal of bonding region 29. In other embodiments, inflation lumens 31, 35 may merge distal of both frangible region 24 and bonding region 29, but distal of distal end 22. As will be described in more detail below, in some embodiments, elongate shaft 12 may include a mixing region where inflation lumens 31, 35 merge in order to facilitate mixing of any media flowing through inflation lumens 31, 35. In such embodiments, the mixing region may be located proximal or distal of frangible region 24.

In the embodiments depicted in Figures 3A and 3B, inflation lumens 31, 35 and guidewire lumen 33 may terminate at ports 6, 7, and/or 8 on hub 17. However, in other embodiments, as depicted in Figures 3C and 3E, catheter 10 may be a single-operator-exchange catheter in which elongate shaft 12 may include a proximal guidewire port 41 at a proximal end of guidewire lumen 33 located distal of hub 17. In the embodiments of Figure 3C, guidewire port 41 may be disposed distal of frangible region 24. In some of these embodiments, region 47 of elongate shaft 12 proximal of guidewire port 41 may constitute a solid material. For instance, region 47 may be a portion of an outer wall of elongate shaft 12. In other embodiments, however, region 47 may be part of inflation lumen 31 and/or 35. For example, the combined cross-sectional area of inflation lumens 31, 35 proximal of guidewire port 41 may be greater than the combined cross-sectional area of inflation lumens 31, 35 distal of guidewire port 41.

Figure 3D depicts a cross-section of frangible region 24 viewed along line E-E. As seen in Figure 3D, where guidewire port 41 is disposed proximal of frangible region 24, elongate shaft 12 may include three lumens, lumens 31, 33, and 35, extending through frangible region 24. In some embodiments, at least a portion of elongate shaft 12 may remain with balloon member 25 after balloon member 25 is detached from elongate shaft 12. In such embodiments, elongate shaft 12 may include one or more frangible features. For instance, in the embodiment of Figure 3D, where elongate shaft 12 is a solid tube, recesses 28 may be formed in the outer wall 13 of elongate shaft 12. Recesses 28 may mechanically weaken the portion of elongate shaft 12 in frangible region 24 such that when pulling or twisting forces are applied to elongate shaft 12, elongate shaft 12 may break or separate along the frangible region 24. In some embodiments, elongate shaft 12 may be extruded to have recesses 28. However in other embodiments, recesses 28 may be formed after extrusion by removing material from elongate shaft 12, such as by cutting or burning with laser ablation.

In the embodiment of Figure 3E, elongate shaft 12 may include a proximal guidewire port 41 at a proximal end of guidewire lumen 33 disposed distal of frangible region 24. For example, guidewire port 41 may be disposed distal of frangible region 24 and proximal of bonding region 29. As with some of the embodiments of Figure 3C, in some of the embodiments of Figure 3E, region 47 of elongate shaft 12 proximal of guidewire port 41 may constitute a solid material. For instance, region 47 may be a portion of an outer wall of elongate shaft 12. In other embodiments, however, region 47 may be part of inflation lumen 31 and/or 35. For example, the combined cross-sectional area of inflation lumens 31, 35 proximal of guidewire port 41 may be greater than the combined cross-sectional area of inflation lumens 31, 35 distal of guidewire port 41.

Figure 3F depicts a cross-section of frangible region 24 viewed along line F-F. As seen in Figure 3F, where guidewire port 41 is disposed distal of frangible region, elongate shaft 12 may include only two lumens, lumens 31 and 35, extending through frangible region 24. Where at least a portion of elongate shaft 12 may remain with balloon member 25 after balloon member 25 is detached from elongate shaft 12, elongate shaft 12 may include one or more frangible features such as recesses 28. In some embodiments where elongate shaft 12 is a solid tube, elongate shaft 12 may be extruded to have recesses 28. However in other embodiments, recesses 28 may be formed after extrusion by removing material from elongate shaft 12, such as by cutting or burning with laser ablation.

Figure 4A depicts a close-up of distal portion 20 of elongate shaft 12 when balloon member 25 is inflated where elongate shaft 12, or a distal region thereof is devoid of a guidewire lumen. For instance, elongate shaft 12 may have no guidewire lumen and may only have a single lumen which is an inflation lumen, or may have multiple (e.g., dual) inflation lumens, such as inflation lumens 31, 35. In such embodiments, elongate shaft 12 may be configured to have different regions of varying stiffness. For instance, elongate shaft 12 may have regions that get progressively less stiff going from the proximal portion of elongate shaft 12 to the distal portion of elongate shaft 12. In such embodiments, the varying stiffness may allow a user to apply pushing forces to elongate shaft 12, yet have distal region 20 of elongate shaft 12 have a low enough stiffness to navigate a potentially tortuous path to the desired occlusion location. In at least some embodiments, elongate shaft 12, or portions of elongate shaft 12, may include one or more reinforcing members to help provide additional stiffness and/or to prevent any of the lumens of elongate shaft 12 from collapsing. For example, the one or more reinforcing member may comprise a coiled wire or a woven layer of material.

In embodiments in accordance with Figure 4A, balloon member 25 may only be bonded to elongate shaft 12 along bonding region 29 and may be disposed around distal end 22 of elongate shaft 12. In these embodiments, distal end 22 of elongate shaft 12 may be sealed. In some embodiments, balloon member 25 may have a waist section 26 that is less compliant than the rest of balloon member 25. Accordingly, when balloon member 25 is inflated, waist section 26 may retain its shape instead of becoming stretched like the other portion of balloon member 25. Waist section 26 may additionally increase the overall diameter elongate shaft 12 in the area of bonding region 29. This increase in diameter may help to bias detachment of balloon member 25 from elongate shaft 12 along frangible region 24.

Figure 4B depicts yet another close-up of distal portion 20 of elongate shaft 12 when balloon member 25 is inflated and includes a guidewire lumen. As depicted in Figure 4B, elongate shaft 12 may include guidewire lumen 33 which extends all the way through balloon member 25. In these embodiments, balloon member 25 may be an annular balloon that is bonded to elongate shaft 12 both along bonding region 29 and along bonding region 30. The distal end of guidewire lumen 33 may be open, thereby allowing catheter 10 to be tracked over a guidewire. In these embodiments, a guidewire may be placed in position within a patient, and then catheter 10 may be placed over the guidewire and maneuvered into position over the guidewire. As depicted in Figure 4B, in some embodiments, ports 93, 95 may be the open ends of inflation lumens 31, 35, instead of being disposed on the side walls of inflation lumens 31, 35. In these embodiments, inflation lumens 31, 35 may terminate within balloon member 25.

In embodiments where guidewire lumen 33 extends all the way through balloon member 25, guidewire lumen 33 may have one or more properties to ensure a seal of guidewire lumen 33 after the guidewire is removed to ensure that balloon member fully occludes the region where it has been positioned. In some embodiments, the walls of guidewire lumen 33, at least in the region within balloon member 25, may be made from a low-durometer material. In such embodiments, when balloon member 25 is inflated, internal pressure from the inflation media may act to press against the walls of guidewire lumen 33 and seal the walls together - thereby preventing fluid from flowing through guidewire lumen 33. In other embodiments, instead of employing a low-durometer material, the walls of guidewire lumen 33 may be thin and have a relatively low stiffness. In a similar manner to if the walls were made from a low-durometer material, when balloon member 25 is inflated, the internal pressure may act to squeeze the walls of guidewire lumen 33 closed. In still other embodiments, the frangible region of elongate shaft 12 may be designed such that when balloon member 25 is detached from elongate shaft 12, the frangible region collapses to close guidewire lumen 33.

Figure 5A depicts an example cross-section of distal region 20 of elongate shaft 12 including an example of frangible region 24 according to some embodiments of the present disclosure. Any internal lumens that elongate shaft 12 may include, for example those shown with respect to Figure 5B, have been removed from Figure 5A for clarity purposes. In the embodiment of Figure 5A, wall 43 of elongate shaft 12 may have a first wall thickness proximal of frangible region 24 (with the proximal direction indicated by arrow P). Thin portion 45 of wall 43 in frangible region 24 may have a second wall thickness, where the second wall thickness is less than the first wall thickness. In some embodiments, elongate shaft 12 may continue extending distal of frangible region 24, and wall 43 may also have the first wall thickness in the region distal of frangible region 24. In other embodiments, wall 43 may have a third wall thickness distal of frangible region 24. In some embodiments, the third wall thickness may be greater than the first wall thickness, however in other embodiments the third wall thickness may be less than the first wall thickness but greater than the second wall thickness.

In some embodiments, thin portion 45 may be made during manufacture by variably thinning wall 43 as elongate shaft 12 is being formed. In other embodiments, thin portion 45 may be formed after elongate shaft 12 has been created by removing material from elongate shaft 12 in frangible region 24 by one or more techniques well known in the art, such as by cutting away the material or using heat to burn away the material - for example with laser ablation. In other embodiments, thin portion 45 may be formed after elongate shaft 12 has been created by further processing, such as stretching or crimping a portion of elongate shaft 12.

Figure 5B depicts an example cross-section of thin portion 45 viewed along line B-B, as depicted in Figure 5A. As seen in Figure 5B, thin portion 45 may have a wall thickness that is thinner than that of wall 43 in other regions of elongate shaft 12. As depicted in Figure 5B, lumens 31, 33, and 35 may all be separate lumens as defined by walls 34,

36, and 38, and reside within lumen 40 of elongate shaft 12. However, in other embodiments, elongate shaft 12 may take a different form where lumens 31, 33, and 35 are not defined by walls 34, 36, and 38. In the embodiment of Figure 5B, walls 34, 36, and 38 may remain the same thickness even through frangible region 24. Figure 5C depicts another example cross-section of thin portion 45 viewed along line B-B. In the embodiment of Figure 5C, the wall thickness of walls 34, 36, and 38 thins, similar to wall 43 of elongate shaft 12, in infrangible region 24. Of course, in other embodiments, less than all of the lumens that reside within elongate shaft 12 may include a thinned portion in frangible region 24. In still other embodiments, one or more of walls 34, 36, and 38 may have thinned portions in regions other than in frangible region 24.

In general, thin portion 45 of wall 43 may be relatively more mechanically weak than the rest of wall 43 of elongate shaft 12. As such, when mechanical stress is applied to catheter 10, such as by pulling or twisting, elongate shaft 12 may preferentially break in frangible region 24, thereby detaching balloon member 25 from the rest of elongate shaft 12. For instance, elongate shaft 12 may break somewhere along thin portion 45. Although, in other examples, elongate shaft 12 may generally, or at least sometimes, break at the interface between thin portion 45 and balloon member 25, or somewhere along balloon member 25. Additionally, where walls 34, 36, and/or 38 do not also have thin portions, when balloon member 25 is detached from elongate shaft 12 and elongate shaft 12 is retracted, lumens 31, 33, and 35 may remain intact and become extracted as elongate shaft 12 is pulled away from balloon member 25. However, where walls 34, 36, and/or 38 do have thin portions, after balloon member 25 is inflated and elongate shaft 12 is retracted, walls 34, 36, and/or 38 may also break along in the region of their thin portions, leaving behind a portion of lumens 31, 33, and 35 as part of detached balloon member 25. Of course, as described, only some of walls 34, 36, and/or 38 may have thin portions. In such embodiments, only those walls that have thin portions may leave behind portions of the lumens, while the other lumens may be entirely extracted as elongate shaft 12 is retracted.

Figure 6 depicts another example cross section of distal region 20 of elongate shaft 12 including an example of frangible region 24. In the example of Figure 6, frangible region 24 includes crimped portions 51. As with the example of Figures 5A-5C, crimped portions 51 may be made during manufacture by variably thinning wall 43 as elongate shaft 12 is being formed. In other examples, crimped portions 51 may be formed after elongate shaft 12 has been created by removing material from elongate shaft 12 in frangible region 24 by one or more techniques well known in the art, such as by cutting away the material or melting or burning away the material - for example with a laser. In still other examples, crimped portions 51 may be created by mechanically compressing, or crimping, elongate shaft 12 in frangible region 24.

As with thin portion 45 in the example of Figure 5A, crimped portions 51 may be relatively more mechanically weak than the rest of elongate shaft 12. As such, when mechanical stress is applied to catheter 10, such as by pulling or twisting, elongate shaft 12 may preferentially break in frangible region 24, along crimped portions 51, thereby detaching balloon member 25 from the rest of elongate shaft 12. Although, in other examples, elongate shaft 12 may generally, or at least sometimes, break at the interface between frangible region 24 and balloon member 25, or somewhere along balloon member 25. Although not shown in Figure 6, any lumens that reside within elongate shaft 12 may also have similar crimped portions 51. In embodiments of Figure 6, the number of lumens residing within elongate shaft 12 that include a frangible feature, and the location of any included frangible features on the included lumens, may be as described with respect to lumens 31, 33, and 35 of Figures 5A-5C.

Figure 7 is a plan view of distal region 20 of elongate shaft 12 including an example of frangible region 24. In the example of Figure 8, frangible region 24 includes slits 53. Slits 53 may be formed after elongate shaft 12 has been created by cutting into elongate shaft 12 in frangible region 24. In some examples, slits 51 may extend all the way through wall 43 of elongate shaft 12. In other examples, slits 51 may only extend through a portion of wall 43.

As with thin portion 45 and crimped portions 51, frangible region 24 including slits 53 may be relatively more mechanically weak than the rest of elongate shaft 12. As such, when mechanical stress is applied to catheter 10, such as by pulling or twisting, elongate shaft 12 may preferentially break in frangible region 24, along slits 53, thereby detaching balloon member 25 from the rest of elongate shaft 12. Although, in other examples, elongate shaft 12 may generally, or at least sometimes, break at the interface between frangible region 24 and balloon member 25, or somewhere along balloon member 25. Although not shown in Figure 7, any lumens that reside within elongate shaft 12 may also have similar slits 53. In embodiments of Figure 7, the number of lumens residing within elongate shaft 12 that include a frangible feature, and the location of any included frangible features on the included lumens, may be as described with respect to lumens 31, 33, and 35 of Figures 5A-5C.

Figure 8 is a plan view of distal region 20 of elongate shaft 12 including an example of frangible region 24. In the example of Figure 8, frangible region 24 includes perforations 55. Perforations 55 may be formed be after elongate shaft 12 has been created by puncturing elongate shaft 12 in frangible region 24 with rods or spikes or the like. Although perforations 55 in Figure 8 are depicted as circular, other examples may have differently shaped perforations. In Figure 8, perforations 55 are depicted as openings in elongate shaft 12. However, in other embodiments, perforations 55 may be small enough that they do not create appreciable openings in elongate shaft 12.

Frangible region 24, including perforations 55, may be relatively more mechanically weak than the rest of elongate shaft 12. As such, when mechanical stress is applied to catheter 10, such as by pulling or twisting, elongate

shaft 12 may preferentially break in frangible region 24, along perforations 55, thereby detaching balloon member 25 from the rest of elongate shaft 12. Although, in other examples, elongate shaft 12 may generally, or at least sometimes, break at the interface between frangible region 24 and balloon member 25, or somewhere along balloon member 25. Although not shown in Figure 8, any lumens that reside within elongate shaft 12 may also have similar perforations 55. In embodiments of Figure 8, the number of lumens residing within elongate shaft 12 that include a frangible feature, and the location of any included frangible features on the included lumens, may be as described with respect to lumens 31, 33, and 35 of Figures 5A-5C.

Figure 9A is a plan view of distal region 20 of elongate shaft 12 including an example of frangible region 24. In the example of Figure 9A, frangible region 24 includes discontinuous recesses 57. Discontinuous recesses 57 may be formed be after elongate shaft 12 has been created by selectively removing material from wall 43 in frangible region 24. Some common techniques know in the art include removing material by cutting or by burning the material away with heat or lasers. In general discontinuous recesses 57 may not extend all the way through wall 43. This can be seen in Figure 9B, which depicts an example cross-sectional shape of frangible region 24, for instance as viewed along line C-C in Figure 9A. Although discontinuous recesses 57 in Figure 9A are depicted as rectangular, other examples may have differently shaped perforations.

Frangible region 24, including discontinuous recesses 57, may be relatively more mechanically weak than the rest of elongate shaft 12. As such, when mechanical stress is applied to catheter 10, such as by pulling or twisting, elongate shaft 12 may preferentially break in frangible region 24, along discontinuous recesses 57, thereby detaching balloon member 25 from the rest of elongate shaft 12. Although, in other examples, elongate shaft 12 may generally, or at least sometimes, break at the interface between frangible region 24 and balloon member 25, or somewhere along balloon member 25. Although not shown in Figures 9A-9B, any lumens that reside within elongate shaft 12 may also have similar discontinuous recesses 57. In embodiments of Figures 9A-9B, the number of lumens residing within elongate shaft 12 that include a frangible feature, and the location of any included frangible features on the included lumens, may be as described with respect to lumens 31, 33, and 35 of Figures 5A-5C.

In the above described examples, frangible region 24 has been depicted disposed on elongate shaft 12 proximal of balloon member 25 and distal of guidewire port 23. However, in other embodiments contemplated by this disclosure, frangible region 24 may be disposed in any of a number of different locations. For example, as depicted in Figure 10A, frangible region 24, including perforations 55 only for exemplary purposes, may be disposed on balloon member 25. Figure 10B depicts an example cross-section of distal region 20 where frangible region 24 is included on balloon member 25. In some embodiments, perforations 55 may extend all the way through balloon member 25 and wall 43 of elongate shaft 12. Although shown as distinct openings in Figure 10B, in other embodiments, perforations 55 may be small enough that they do not create any appreciable opening or connection between the interior of balloon member 25 and/or elongate shaft 12 to the exterior of balloon member 25. Again, any distinct lumens residing within elongate shaft 12 may or may not also have such frangible features, as described with respect to lumens 31, 33, and 35 of Figures 5A-5C.

Figure 11 depicts yet another example cross-section of distal portion 20 of elongate shaft 12. In the embodiment of Figure 11, elongate shaft 12 may not have a frangible region. Rather, balloon member 25 may be attached to elongate shaft 12 using adhesive 61. In these embodiments, adhesive 61 may be a soluble adhesive or a hydrophilic coating. For instance, adhesive 61 may include polyvinylpyrrolidone (PVP), polyethylene glycol (PEO), polyvinyl alcohol (PVA), sodium alginate, chitosan, polyacrylic acid, and/or polyacrylamide, or other suitable adhesives or hydrophilic coatings.

In these embodiments, adhesive 61 may temporarily attach balloon member 25 to elongate shaft 12. Such soluble adhesives or hydrophilic coatings may be water soluble, or at least soluble in the aqueous environment of blood. After a sufficient amount of time exposed to the patient's blood or other solvent, the bond between balloon member 25 and elongate shaft 12 may weaken. In some instances, balloon member 25 may separate from elongate shaft 12 after exposure to blood or another solvent without external forces being applied. However, in other instances, some external force, such as a pulling or twisting of catheter 10, may be used to separate balloon member 25 from elongate shaft 12. In other embodiments, adhesive 61 may be exposed to a lumen of elongate shaft 12. In such embodiments, an appropriate solvent (for example, water) may be injected in the lumen exposed to adhesive 61 to weaken the bond between balloon member 25 and elongate shaft 12.

In the above described embodiments, elongate shaft 12 may be formed according to techniques known in the art. Balloon member 25 may then be adhesively or thermally attached to distal portion 20 of elongate shaft 12. Balloon member 25 may be extruded using a compliant, low durometer, elastomeric material, such as silicone, thermoplastic polyurethane (TPU), SIBS (poly styrene-isobutylene-styrene block copolymer), polyurethane, SEBS styrene ethylene butylene styrene block copolymer, other styrenic block copolymers, or other suitable materials. Once balloon member 25 has been attached to elongate shaft 12, a frangible region in accordance with this disclosure may be formed to create a detachable site. Although, in other embodiments, it is possible the frangible region is created on elongate shaft 12 before balloon member 25 is attached. In some additional embodiments, instead of being extruded onto elongate shaft 12, balloon member 25 may be a separate tube section that is attached to elongate shaft 12 along distal portion 20. In such embodiments, balloon member 25 may be compression-fitted, heat-bonded, laser-welded or otherwise attached

to elongate shaft 12.

However, in still additional embodiments, balloon member 25 and elongate shaft 12 may be formed in an integral manner. Figures 12A and 12B depict example cross-sections of distal portion 20 of elongate shaft 12 where elongate shaft 12 and balloon member 25 have been formed integrally, where balloon member 25 is in an uninflated state (Figure 12A) and an inflated state (Figure 12B). As elongate shaft 12 is formed, wall 43 may be variably thinned to produce balloon 25. Once elongate shaft 12 and balloon member 25 have been formed, a frangible region may be created on elongate shaft 12 or balloon member 25, as desired, for instance in accordance with any of the techniques discussed with respect to Figures 3A-11.

Figures 13A-13D depict how catheter 10 may operate to occlude a cavity. In one embodiment, balloon member 25 may be guided to a location within vasculature 101 of a patient to occlude aneurysm 103. In some instances, a guidewire (not shown) may be guided through vasculature 101 of the patient to the desired area, e.g. aneurysm 103. Once the guidewire is in place, catheter 10 may be placed over the guidewire and threaded along the guidewire to arrive at aneurysm 103. After balloon member 25 is positioned in aneurysm 103, as shown in Figure 13A, balloon member 25 may be inflated, for example by injecting various gases, fluids, or foam-forming chemistries. Figure 13B shows balloon member 25 partially inflated.

In some embodiments, balloon member 25 may further include biologically safe adhesive 71 disposed on the outer wall of balloon member 25. Biologically safe adhesive 71 may act as a tissue sealant or a mucosal adhesive and may be safe for use within a human body. Some examples of biologically safe adhesive 71 include hydrogels comprised of polymers. One example hydrogel is a copolymer of vinyl pyrrolidone, acrylic acid, and N-hydroxysuccinimide. An example structure of such a copolymer is shown below:

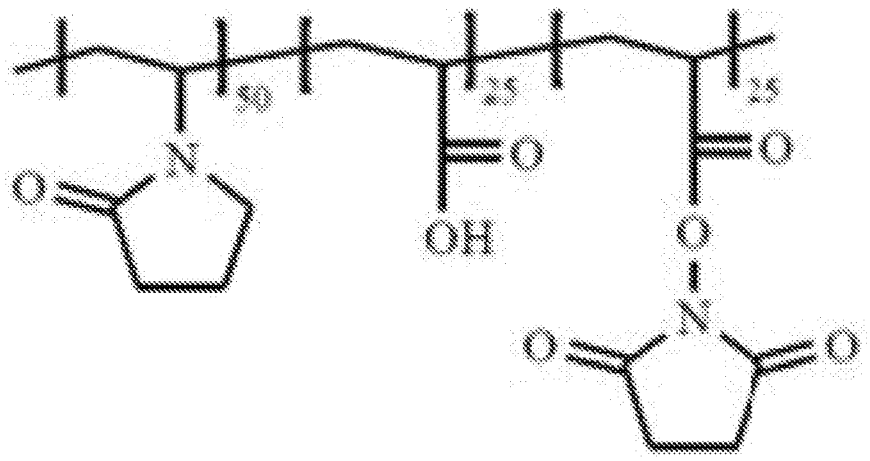

Other example biologically safe adhesives include biomimetic adhesives comprising synthetic hydrogels (PEO as one example) modified with catechol functionality (mussel-like adhesives), and cross-linked polyacrylic acid and copolymers. Once balloon member 25 is disposed within aneurysm 103 fully expanded, biologically safe adhesive 71 may operate to secure balloon member 25 within aneurysm 103.

As balloon member 25 is inflated, balloon member 25 may tend to conform to the shape of aneurysm 103. Once fully inflated, as in Figure 13C, a force may be applied to catheter 10, such as a retracting force or a twisting force. Balloon member 25 may be retained within aneurysm 103 in the face of the force applied to catheter 10 due one or more retaining forces. For example, aneurysm 103 may have a relatively small neck, which may operate to prevent inflated balloon member 25 from being pulled out of aneurysm 103. Additionally, or alternatively, in examples where balloon member 25 includes biologically safe adhesive 71, biologically safe adhesive 71 may secure balloon member 25 to the walls of aneurysm 103. The securing force provided by biologically safe adhesive 71 may then operate to prevent balloon member 25 from being pulled out of aneurysm 103. The opposing force on catheter 10 and the retention forces on balloon member 25 may be sufficient to break elongate shaft 12 along, or near, frangible region 24. This breakage separates balloon member 25 from the rest of elongate shaft 12, leaving balloon member 25 disposed within aneurysm 103, as depicted in Figure 13D.

Figures 14A-14C depict balloon member 25 being positioned and deployed in a collateral vessel. For instance, elongate shaft 12 may be guided into branch 175. Once in position, balloon member 25 may be inflated, as depicted in Figure 14B. When balloon member 25 is inflated to the desired level, elongate shaft 12 may be retracted, and balloon member 25 may be detached from the rest of elongate shaft 12, as in Figure 14C.

In some embodiments, balloon member 25 may be inflated with a polymer material, which may be a foam-forming polymer material. The polymer material reactants may individually initially have a sufficiently low viscosity to

allow flow through elongate shaft 12 and into balloon member 25. However, once the polymer material reactants have mixed, possibly along with application of heat or electricity, the polymer material reactants may harden into a solid polymer material or expanded foam. The solid polymer material or expanded foam may help to prevent blood from flowing into balloon member 25, aneurysm 103, and/or branch 175. In other examples, the solidified polymer material or expanded foam may allow for some perfusion of blood into balloon member 25, which may result in a clot forming within balloon member 25. In these embodiments, the solidified polymer material or expanded foam may act to block blood from flowing into aneurysm 103, or into/out of branch 175.

In embodiments where the polymer material reactants are foam-forming reactants, the foam-forming reactants may be liquid. Once the reactants are mixed together, the liquid reactants may begin to expand in a foaming fashion and eventually harden. For instance, in some embodiments, the interior of balloon member 25 may be coated with a super absorbant polymer (SAP) such as lightly cross-linked poly sodium acrylate. When the balloon is inflated with water, the SAP swells resulting in gelation of the inflation media. In other embodiments, an aqueous solution (e.g. 1% solids) of polyacrylic acid may be injected into the balloon through a first lumen and an aqueous solution of base (e.g. NaOH or sodium bicarbonate) may be injected through a second lumen. Mixing of the two solutions may result in neutralization of the polyacrylic acid and forming gelled polysodium acrylate. In still other embodiments, a foam may be formed using a reaction according to equation (1).

$$\text{isocyanate} + \text{polyol} + \text{water} = \text{polyurethane} + \text{CO2} = \text{polyurethane foam} \quad (1)$$

Example isocyanates that may be used include hexamethyline diisocyanate (HDI), toluene diisocyanate (TDI), xylene diisocyanate, methylene diphenyl diisocyanate (MDI), lysine diisocyanate, and isophorone diisocyanate. Example polyols that may be used include polyether, polybutadiene polyols, polysiloxane polyols, polypropylene glycols (PPG), and polyethylene glycols (PEG).

In general, by utilizing different reactants or reactants in varying proportions, foams having specific, differing properties may be formed. For instance, various foams used to inflated balloon member 25 may have pore size ranging from 5-500 micrometers and may have anywhere between 10-10,000 cells. Further, the stiffness of the foam may be controllable based on the types and quantities of the reactants used. In some embodiments, radiopaque materials may be added to balloon member, either before, during, or after inflation to make balloon member 25 easier to see on various medical imaging machines.

As described previously with respect to Figures 3A-3B, in some embodiments, elongate shaft 12 may include a mixing region where two inflation lumens merge. The mixing region may help to evenly mix reactants used in a foam-forming reaction to inflate balloon member 25. Figures 15-17 depict different embodiments of a mixing region.

Figure 15 is a cross-section of distal region 20 of elongate shaft 12 and an example mixing region 73. In the example of Figure 15, mixing region 73 is situated at the merging of inflation lumens 31, 35 into lumen 29. Mixing region 73 comprises one or more barriers 81 that extend from inner wall 83 of elongate shaft 12 and/or from wall 36 that defines guidewire lumen 33 at least part-way into the lumen in mixing region 73. In some examples, barriers 81 extend half-way, two-thirds, or three-quarters, or any other suitable distance into the lumen in mixing region 73. Consecutive barriers 81 may extend into the lumen in mixing region 73 from different directions to create a tortuous flow path to encourage mixing between the gases or fluids flowing through lumens 31 and 35. Although mixing region 73 is depicted proximal (with the proximal direction being indicated by arrow P) of frangible region 24, in other embodiments, mixing region 73 may be located in other regions of elongate shaft 12. For instance, in other embodiments, mixing region 73 may be located distal (with the distal direction being indicated by arrow D) of frangible region 24. Although not shown in Figure 15, one or more inflation ports (for instance, inflation ports 93, 95 as depicted in Figures 3A-3B) may place lumen 29 in communication with the interior of balloon member 25, such that inflation media that flow through lumens 31, 35, and 29 may inflate balloon member 25.

Figure 16 is a cross-section of distal region 20 of elongate shaft 12 and another example mixing region 73. In the example of Figure 16, mixing region 73 is situated at the merging of inflation lumens 31, 35 into lumen 29. Mixing region 73 comprises one or more first barriers 85 and one or more second barriers 87. First barriers 85 may be circular or other shaped barriers that are situated within the center of the lumen in mixing region 73. First barriers 85 may be connected to inner wall 83 of elongate shaft 12 and from wall 36 of guidewire lumen 33 by small connecting arms spaced around first barriers 85. In this manner, first barriers 85 allow for flow of liquids or gas around toward walls 43 and 36. Second barriers 87, on the other hand, may generally extend from inner wall 83 and wall 36 toward the center of the lumen of mixing region 73. For instance, second barriers 87 may be solid membranes filling the entire lumen of mixing region 73 except for a hole in their centers. In this manner, fluid may flow through second barriers 87 through the center of the lumen of mixing region 73. Alternating first barriers 85 and second barriers 87 in this manner may create a tortuous flow path to encourage mixing between the gases or fluids flowing through lumens 31 and 35.

Although mixing region 73 is depicted proximal (with the proximal direction being indicated by arrow P) of frangible region 24, in other embodiments, mixing region 73 may be located in other regions of elongate shaft 12. For instance, in other embodiments, mixing region 73 may be located distal (with the distal direction being indicated by arrow D) of frangible region 24. Additionally, although not shown in Figure 16, one or more inflation ports (for instance, inflation ports 93, 95 as depicted in Figures 3A-3B) may place lumen 29 in communication with the interior of balloon member 25, such that inflation media that flow through lumens 31, 35, and 29 may inflate balloon member 25.

Figure 17 is a cross-section of distal region 20 of elongate shaft 12 and yet another example mixing region 73. In the example of Figure 17, mixing region 73 comprises static helical mixer 89. Static helical mixer 89 creates a tortuous path for liquids or gases or flowing through lumens 31 and 35 to encourage mixing as during flowing along static helical mixer 89. Although mixing region 73 is depicted proximal (with the proximal direction being indicated by arrow P) of frangible region 24, in other embodiments, mixing region 73 may be located in other regions of elongate shaft 12. For instance, in other embodiments, mixing region 73 may be located distal (with the distal direction being indicated by arrow D) of frangible region 24. Additionally, although not shown in Figure 17, one or more inflation ports (for instance, inflation ports 93, 95 as depicted in Figures 3A-3B) may place lumen 29 in communication with the interior of balloon member 25, such that inflation media that flow through lumens 31, 35, and 29 may inflate balloon member 25.

Figure 18 is a flow diagram of an illustrative method for forming a medical device, for example catheter 10 as depicted with respect to Figure 1. Although Figure 18 will be described with respect to catheter 10 and Figure 1, the method of Figure 18 may be used to form other medical devices. A first step may comprise forming a catheter shaft having a proximal end and a distal end, such as in step 201. In some examples, the catheter shaft may include a plurality of lumens extending through at least a portion of the catheter shaft. For example, the plurality of lumens may be arranged as depicted in any of Figures 2A-2C. However, in other examples, the plurality of lumens may be formed or arranged differently than depicted in Figures 2A-2C. Additionally, in some examples, the catheter shaft may terminate in a balloon member, such as balloon member 25. Next, a distal portion of the catheter shaft may be weakened to create a frangible region, as in step 203. For example, the frangible region may comprise any of the frangible regions described above with respect to other Figures and be formed according to any of the disclosed techniques.

Those skilled in the art will recognize that the present disclosure may be manifested in a variety of forms other than the specific embodiments described and contemplated herein. Specifically, the various features described with respect to the various embodiments and figures should not be construed to be applicable to only those embodiments and/or figures. Rather, each described feature may be combined with any other feature in various contemplated embodiments, either with or without any of the other features described in conjunction with those features. Accordingly, departure in form and detail may be made without departing from the scope of the present disclosure as described in the appended claims.

## Claims

1. A medical device comprising:

   a catheter shaft (12) extending from a proximal end to a distal end, the catheter shaft (12) including a plurality of lumens (31, 33, 35) extending through at least a portion of the catheter shaft (12); and
   a balloon member (25) disposed proximate the distal end of the catheter shaft (12),
   wherein the catheter shaft (12) comprises a frangible portion (24) disposed proximate the distal end of the catheter shaft (12) for detaching the balloon member (25) from the catheter shaft (12) **characterised in that** at least two of the plurality of lumens (31, 33, 35) merge into a single lumen which opens into the balloon member (25).

2. The medical device of claim 1, wherein the catheter shaft (12) has a first wall thickness proximal of the frangible portion (24), and the frangible portion (24) has a second wall thickness, and wherein the second wall thickness is less than the first wall thickness.

3. The medical device of any of claims 1-2, wherein the frangible portion (24) comprises one or more perforations through the catheter shaft (12).

4. The medical device of any of claims 1-3, wherein the frangible portion (24) comprises one or more discontinuous recesses in an outer wall of the catheter shaft (12).

5. The medical device of any of claims 1-4, wherein the frangible portion (24) is disposed proximal of the balloon member (25).

6. The medical device of any of claims 1-4, wherein the frangible portion (24) is disposed on the balloon member (25).

7. The medical device of any of claims 1-6, the catheter shaft (12) further comprises a mixing region (73) disposed proximate the distal end of the catheter shaft (12).

8. The medical device of claim 7, wherein the mixing region (73) comprises one or more barriers extending part way into one of the plurality of lumens (31, 33, 35).

9. The medical device of any of claims 7-8, wherein the mixing region (73) comprises a static helical mixer (89).

10. The medical device of any of claims 1-9, further comprising a biologically safe adhesive disposed on an outside of the balloon member (25).

11. The medical device of any of claims 1-10, wherein the balloon member (25) is a separate component from the catheter shaft (12).

12. The medical device of any of claims 1-10, wherein the balloon member (25) is integral with the catheter shaft (12).

13. The medical device of any of claims 1-11, wherein the balloon member (25) is connected to the catheter shaft (12) with an adhesive that is soluble in an aqueous environment of blood.

14. The medical device of any of claims 1-13, wherein at least one of the plurality of lumens (31, 33, 35) is a guidewire lumen.

**Patentansprüche**

1. Medizinprodukt, das aufweist:

   einen Katheterschaft (12), der sich von einem proximalen Ende zu einem distalen Ende erstreckt, wobei der Katheterschaft (12) mehrere Lumen (31, 33, 35) aufweist, die sich durch mindestens einen Abschnitt des Katheterschafts (12) erstrecken; und
   ein Ballonteil (25), das nahe dem distalen Ende des Katheterschafts (12) angeordnet ist,
   wobei der Katheterschaft (12) einen abtrennbaren Abschnitt (24), der nahe dem distalen Ende des Katheterschafts (12) angeordnet ist, zum Abtrennen des Ballonteils (25) vom Katheterschaft (12) aufweist,
   **dadurch gekennzeichnet, dass** sich mindestens zwei der mehreren Lumen (31, 33, 35) zu einem einzelnen Lumen vereinigen, das sich in das Ballonteil (25) öffnet.

2. Medizinprodukt nach Anspruch 1, wobei der Katheterschaft (12) eine erste Wanddicke proximal des abtrennbaren Abschnitts (24) hat und der abtrennbare Abschnitt (24) eine zweite Wanddicke hat und wobei die zweite Wanddicke kleiner als die erste Wanddicke ist.

3. Medizinprodukt nach Anspruch 1 oder 2, wobei der abtrennbare Abschnitt (24) eine oder mehrere Perforationen durch den Katheterschaft (12) aufweist.

4. Medizinprodukt nach einem der Ansprüche 1 bis 3, wobei der abtrennbare Abschnitt (24) eine oder mehrere diskontinuierliche Aussparungen in einer Außenwand des Katheterschafts (12) aufweist.

5. Medizinprodukt nach einem der Ansprüche 1 bis 4, wobei der abtrennbare Abschnitt (24) proximal des Ballonteils (25) angeordnet ist.

6. Medizinprodukt nach einem der Ansprüche 1 bis 4, wobei der abtrennbare Abschnitt (24) an dem Ballonteil (25) angeordnet ist.

7. Medizinprodukt nach einem der Ansprüche 1 bis 6, wobei der Katheterschaft (12) ferner einen Mischbereich (73) aufweist, der nahe dem distalen Ende des Katheterschafts (12) angeordnet ist.

8. Medizinprodukt nach Anspruch 7, wobei der Mischbereich (73) eine oder mehrere Sperren aufweist, die sich teilweise

in eines der mehreren Lumen (31, 33, 35) erstrecken.

**9.** Medizinprodukt nach Anspruch 7 oder 8, wobei der Mischbereich (73) einen statischen Schneckenmischer (89) aufweist.

**10.** Medizinprodukt nach einem der Ansprüche 1 bis 9, ferner mit einem biologisch unbedenklichen Kleber, der auf einer Außenseite des Ballonteils (25) angeordnet ist.

**11.** Medizinprodukt nach einem der Ansprüche 1 bis 10, wobei das Ballonteil (25) eine vom Katheterschaft (12) getrennte Komponente ist.

**12.** Medizinprodukt nach einem der Ansprüche 1 bis 10, wobei das Ballonteil (25) einteilig mit dem Katheterschaft (12) ist.

**13.** Medizinprodukt nach einem der Ansprüche 1 bis 11, wobei das Ballonteil (25) mit dem Katheterschaft (12) mit einem Kleber verbunden ist, der in einer wässrigen Blutumgebung löslich ist.

**14.** Medizinprodukt nach einem der Ansprüche 1 bis 13, wobei mindestens eines der mehreren Lumen (31, 33, 35) ein Führungsdrahtlumen ist.

## Revendications

**1.** Dispositif médical comprenant :

une tige de cathéter (12) qui s'étend entre une extrémité proximale et une extrémité distale, la tige de cathéter (12) comprenant une pluralité de lumières (31, 33, 35) qui s'étendent dans au moins une partie de la tige de cathéter (12) ; et
un élément de ballonnet (25) disposé près de l'extrémité distale de la tige de cathéter (12),
dans lequel la tige de cathéter (12) comprend une partie frangible (24) disposée près de l'extrémité distale de la tige de cathéter (12) afin de détacher l'élément de ballonnet (25) de la tige de cathéter (12), **caractérisé en ce qu'**au moins deux de la pluralité de lumières (31, 33, 35) fusionnent en une seule lumière qui s'ouvre dans l'élément de ballonnet (25).

**2.** Dispositif médical selon la revendication 1, dans lequel la tige de cathéter (12) possède une épaisseur de première paroi proche de la partie frangible (24), et la partie frangible (24) possède une seconde épaisseur de paroi, et dans lequel la seconde épaisseur de paroi est inférieure à la première épaisseur de paroi.

**3.** Dispositif médical selon l'une quelconque des revendications 1 ou 2, dans lequel la partie frangible (24) comprend une ou plusieurs perforations dans la tige de cathéter (12).

**4.** Dispositif médical selon l'une quelconque des revendications 1 à 3, dans lequel la partie frangible (24) comprend un ou plusieurs renfoncements discontinus dans une paroi externe de la tige de cathéter (12).

**5.** Dispositif médical selon l'une quelconque des revendications 1 à 4, dans lequel la partie frangible (24) est disposée près de l'élément de ballonnet (25).

**6.** Dispositif médical selon l'une quelconque des revendications 1 à 4, dans lequel la partie frangible (24) est disposée sur l'élément de ballonnet (25).

**7.** Dispositif médical selon l'une quelconque des revendications 1 à 6, la tige de cathéter (12) comprenant en outre une zone de mélange (73) disposée près de l'extrémité distale de la tige de cathéter (12).

**8.** Dispositif médical selon la revendication 7, dans lequel la zone de mélange (73) comprend une ou plusieurs barrières qui s'étendent en partie dans l'une de la pluralité de lumières (31, 33, 35).

**9.** Dispositif médical selon l'une quelconque des revendications 7 à 8, dans lequel la zone de mélange (73) comprend un mélangeur hélicoïdal statique (89).

**10.** Dispositif médical selon l'une quelconque des revendications 1 à 9, comprenant en outre un adhésif biologiquement sûr disposé sur un extérieur de l'élément de ballonnet (25).

**11.** Dispositif médical selon l'une quelconque des revendications 1 à 10, dans lequel l'élément de ballonnet (25) est un composant distinct de la tige de cathéter (12).

**12.** Dispositif médical selon l'une quelconque des revendications 1 à 10, dans lequel l'élément de ballonnet (25) fait partie intégrante de la tige de cathéter (12).

**13.** Dispositif médical selon l'une quelconque des revendications 1 à 11, dans lequel l'élément de ballonnet (25) est relié à la tige de cathéter (12) avec un adhésif qui est soluble dans un environnement sanguin aqueux.

**14.** Dispositif médical selon l'une quelconque des revendications 1 à 13, dans lequel au moins l'une de la pluralité de lumières (31, 33, 35) est une lumière de fil de guidage.

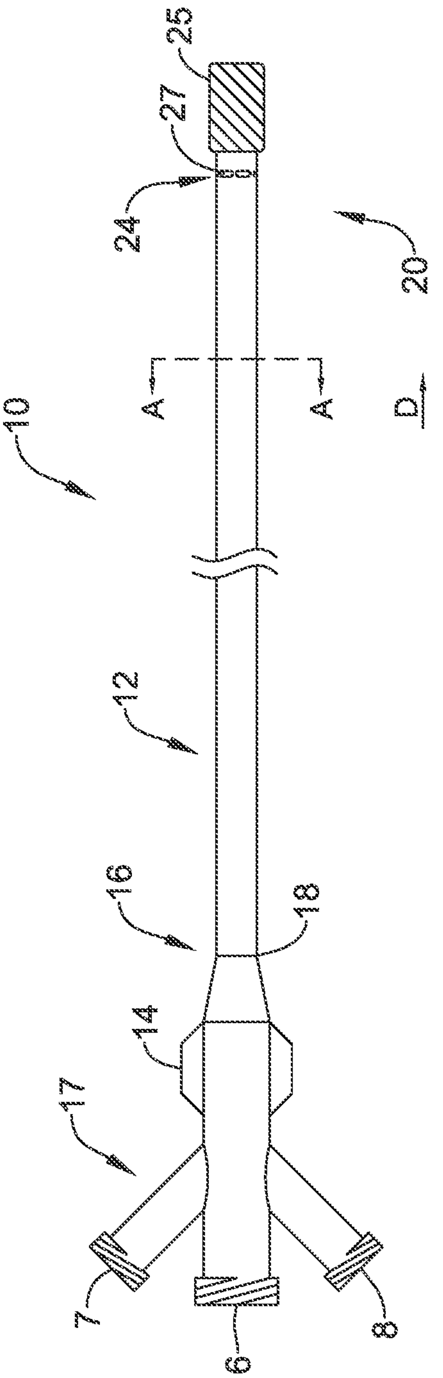


FIG. 1

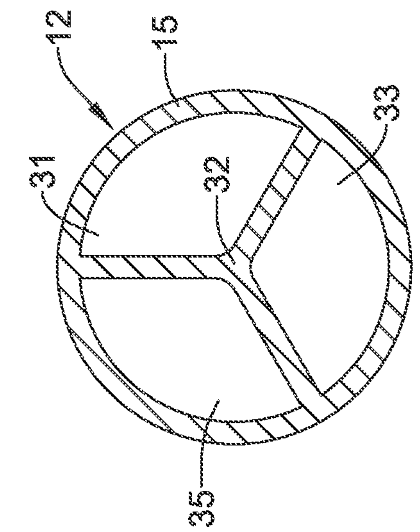


FIG. 2B

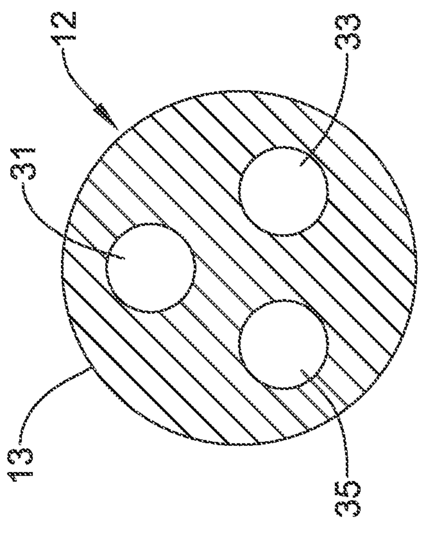


FIG. 2A

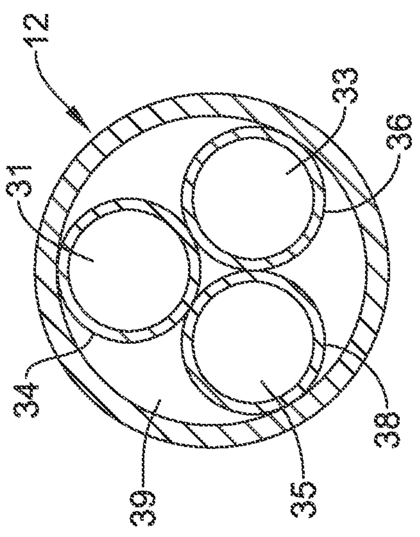


FIG. 2C

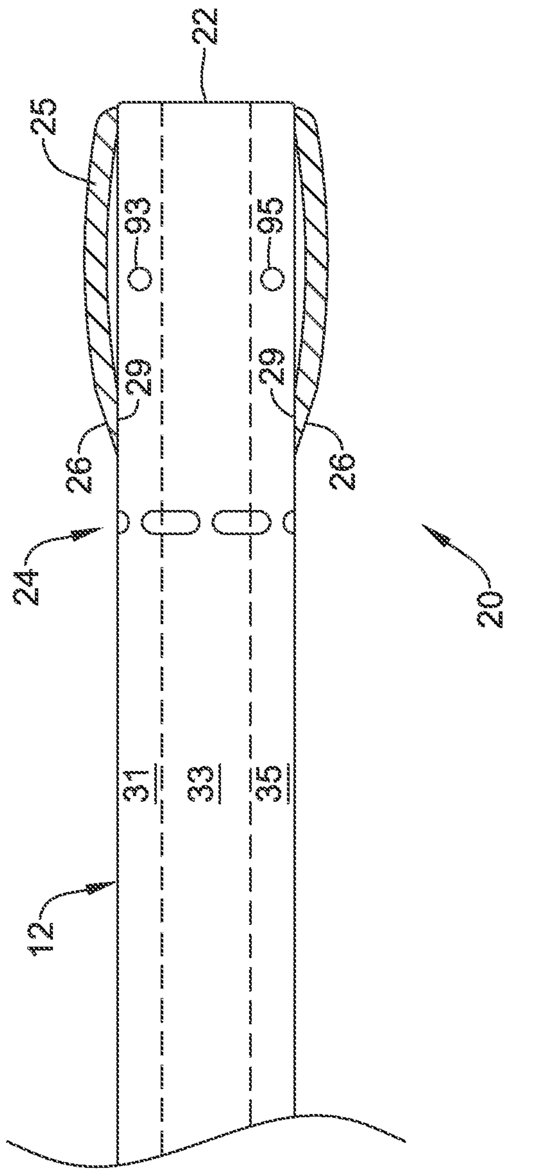


FIG. 3A

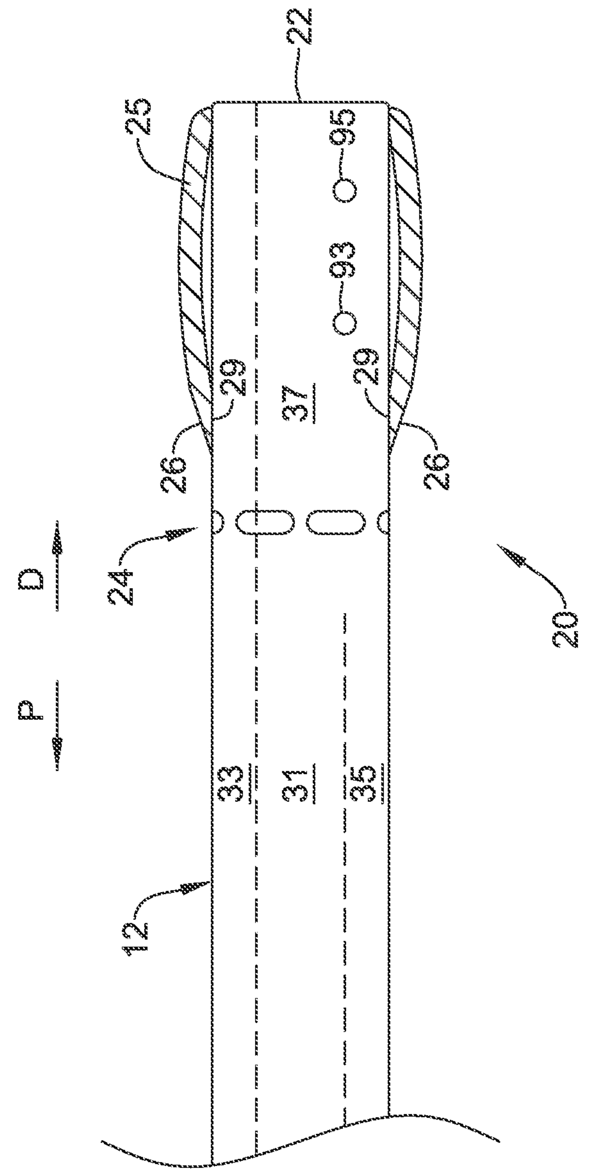


FIG. 3B

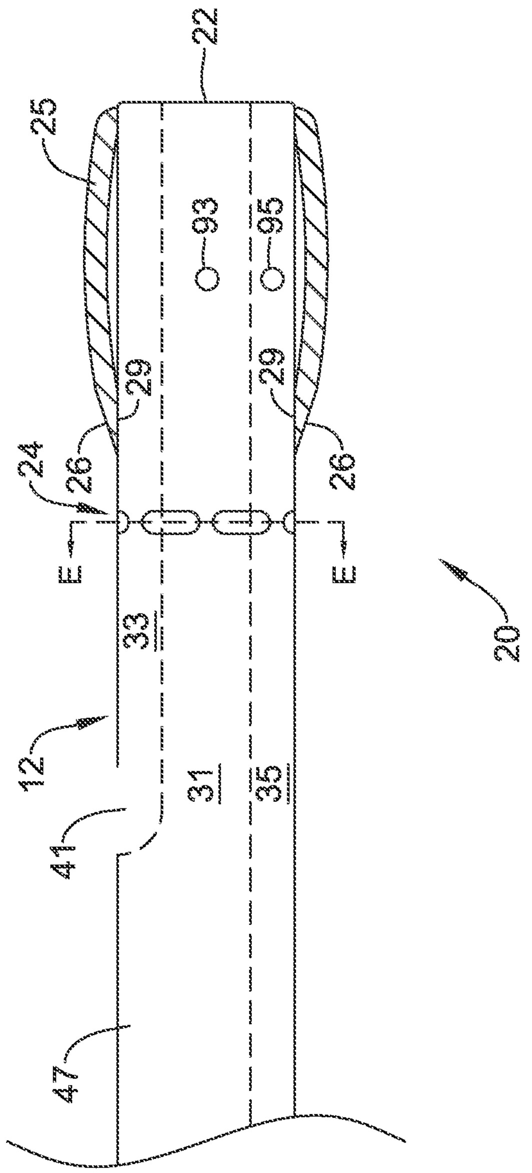


FIG. 3C

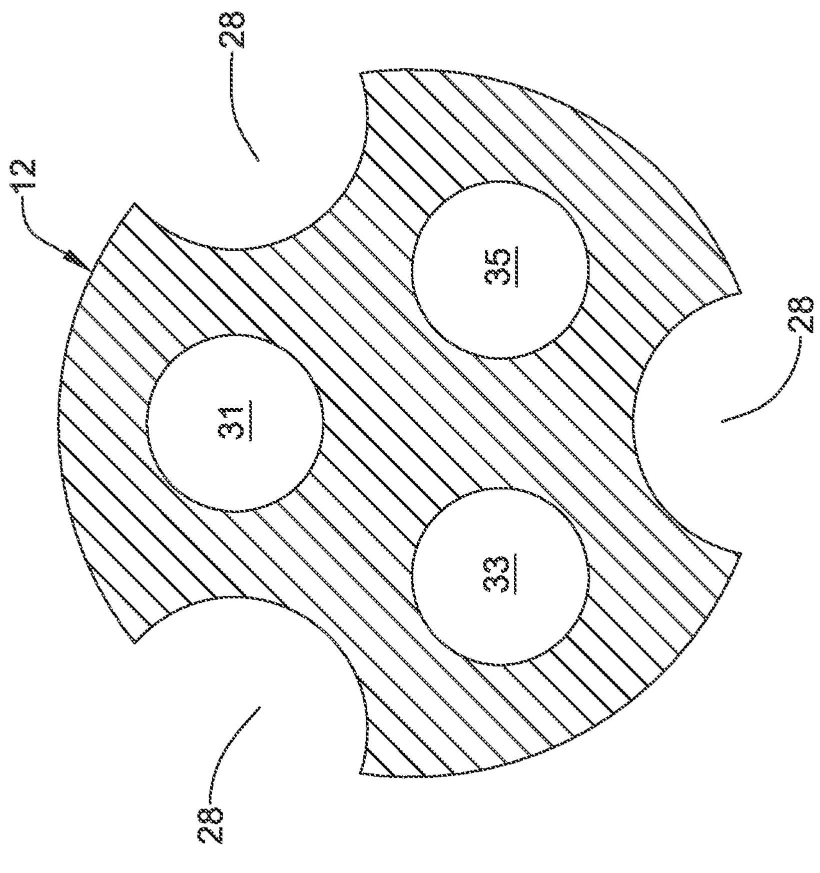


FIG. 3D

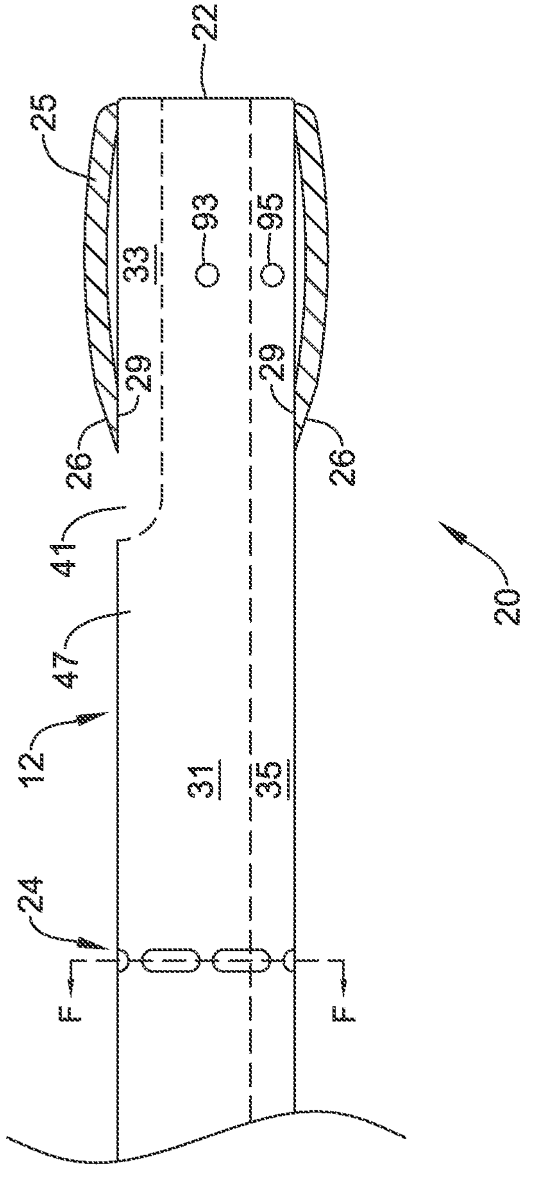


FIG. 3E

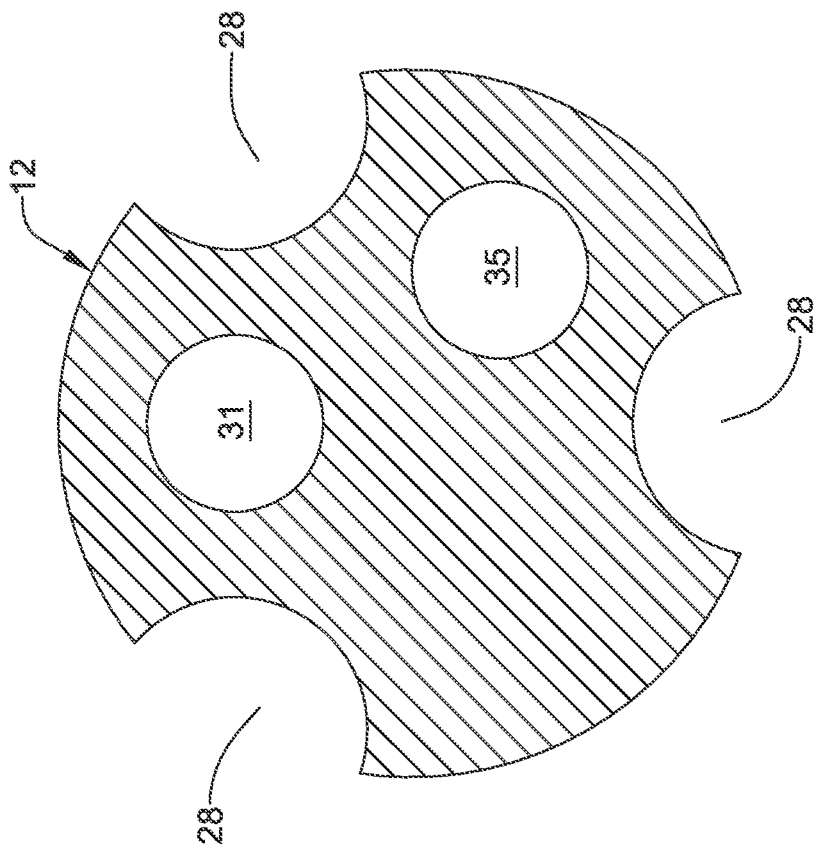


FIG. 3F

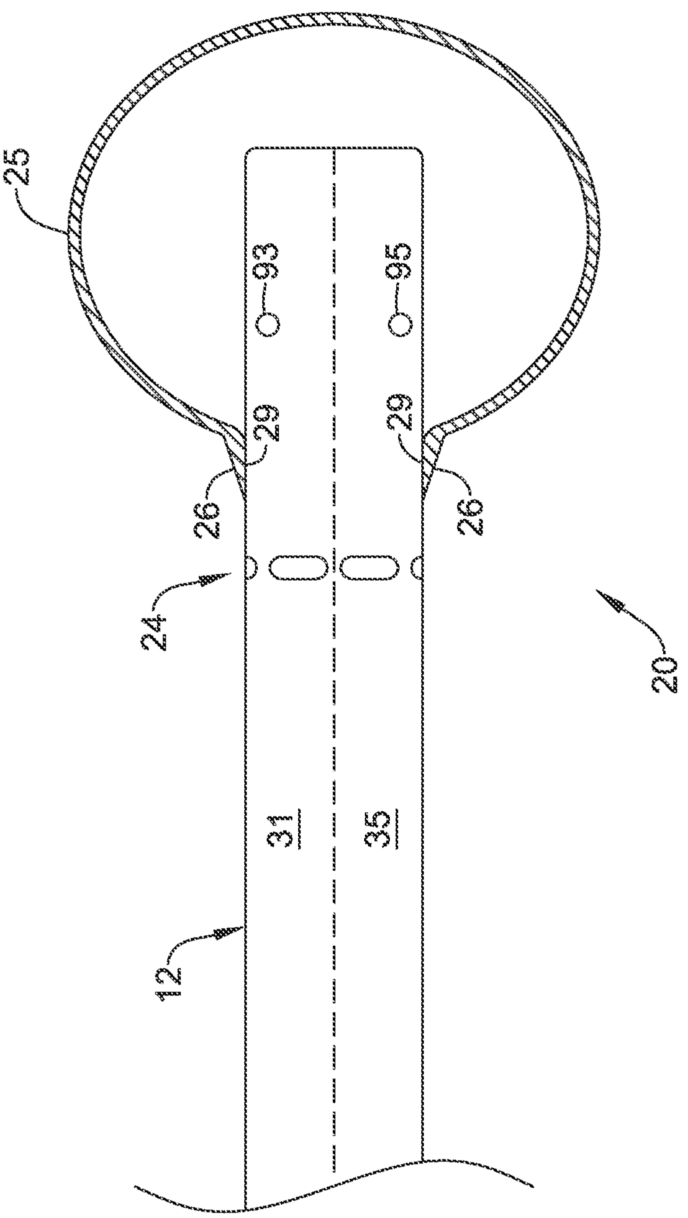


FIG. 4A

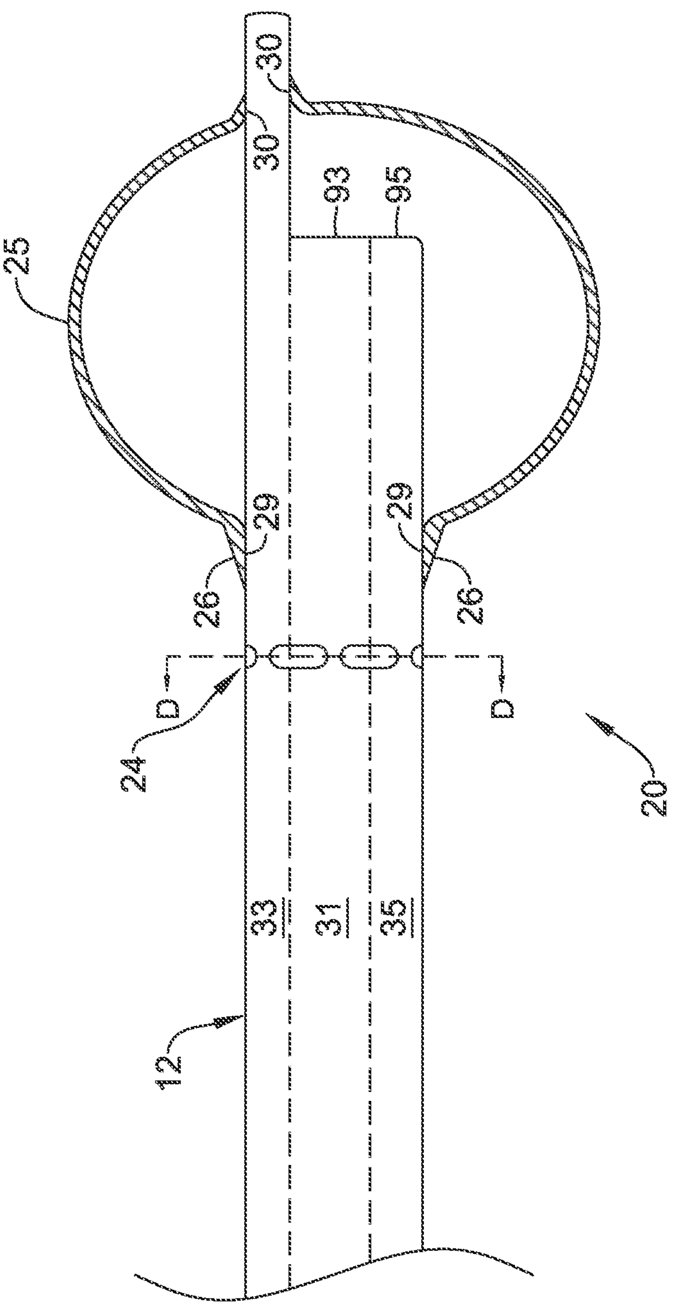


FIG. 4B

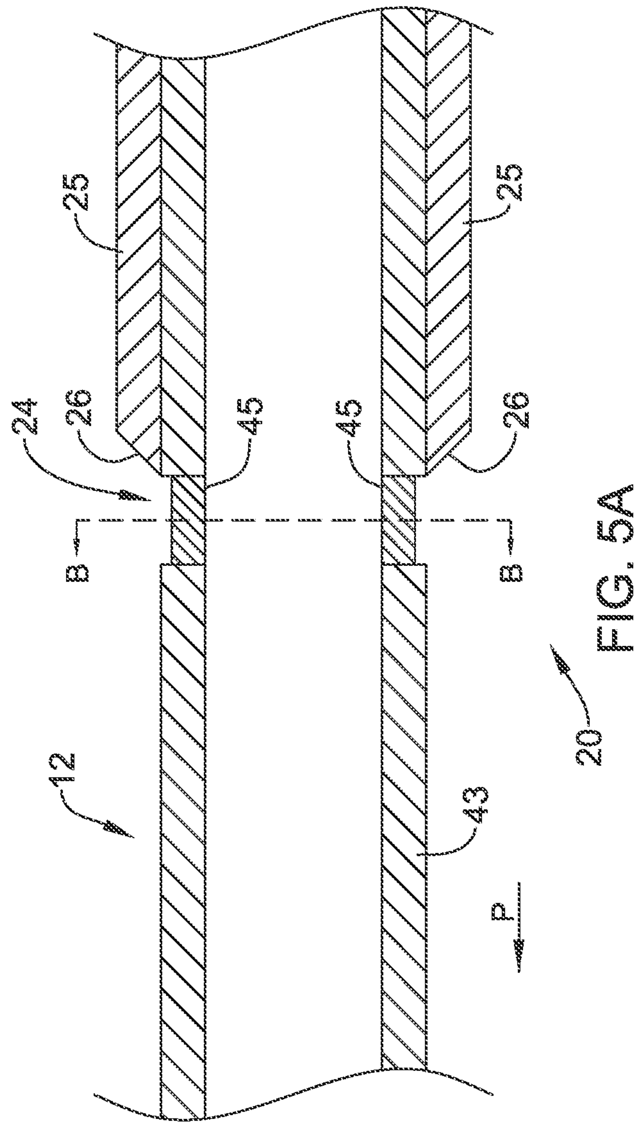


FIG. 5A

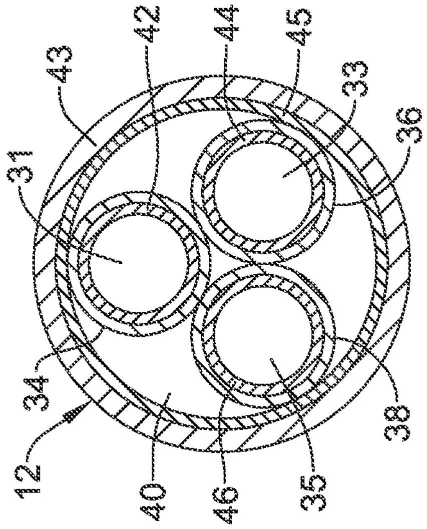


FIG. 5C

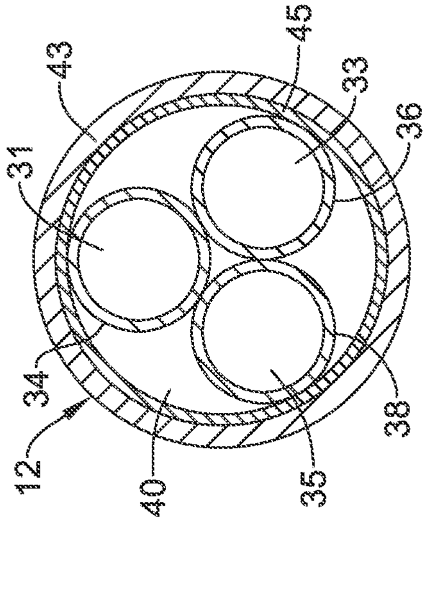


FIG. 5B

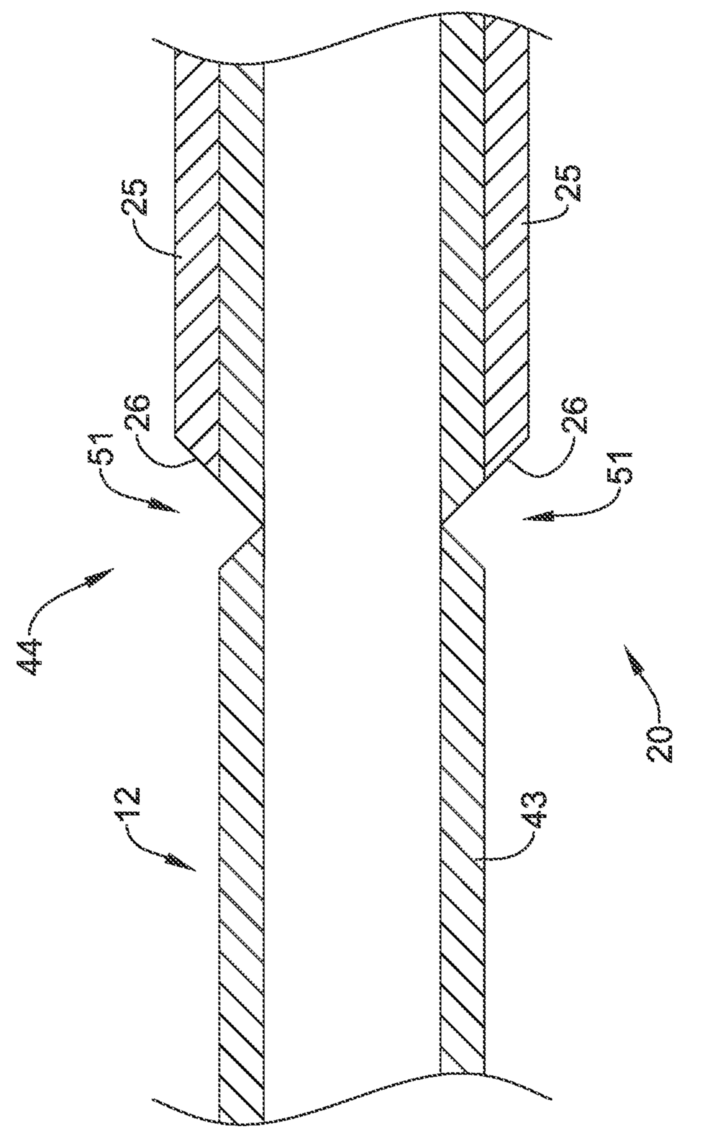


FIG. 6

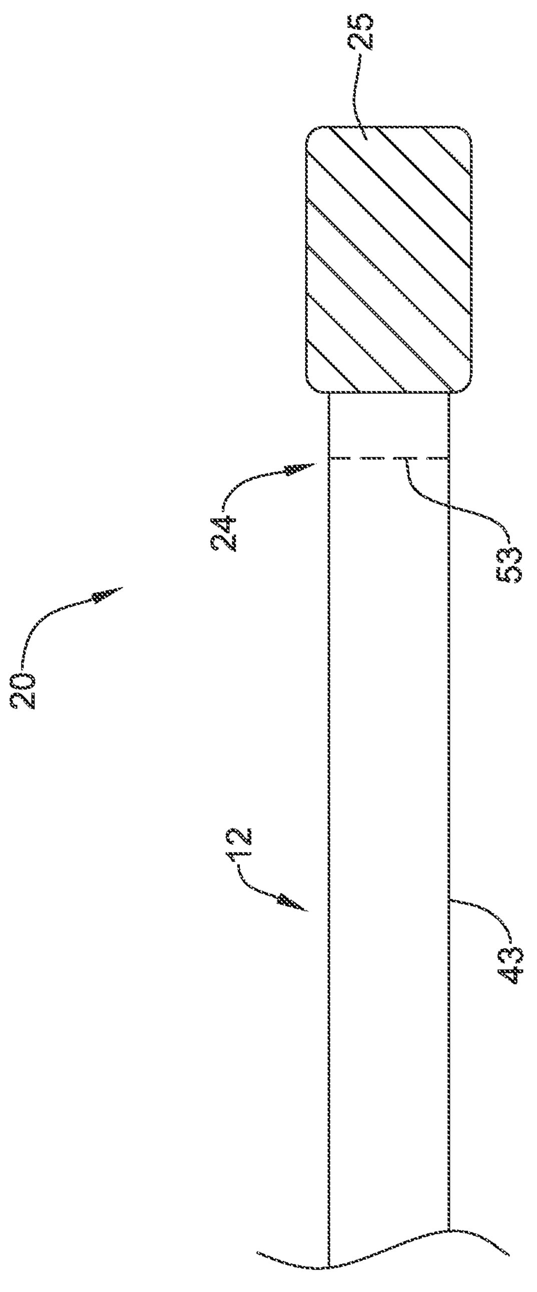


FIG. 7

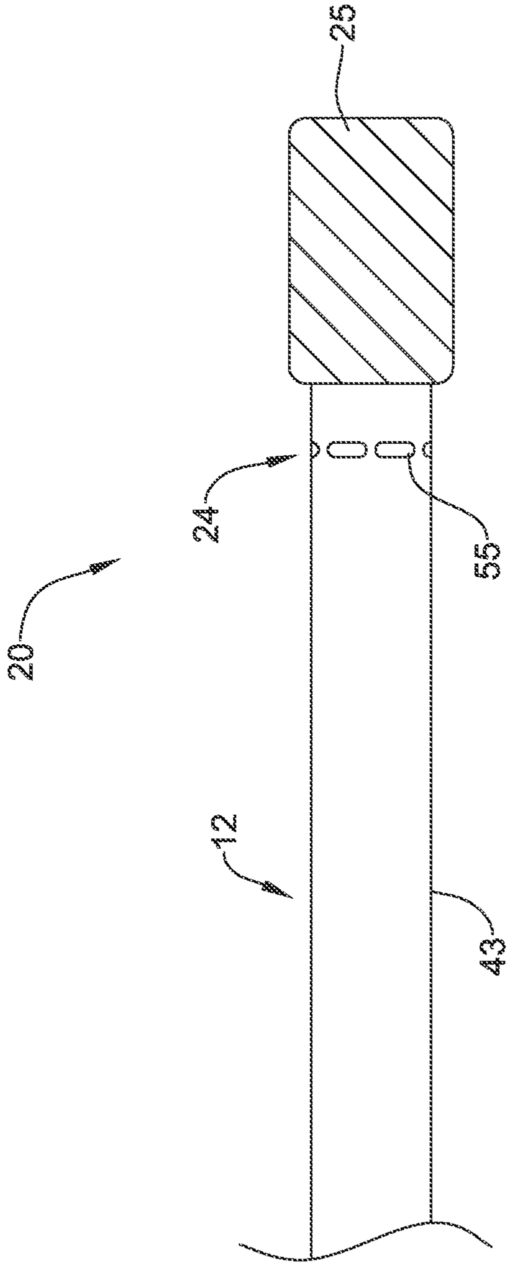


FIG. 8

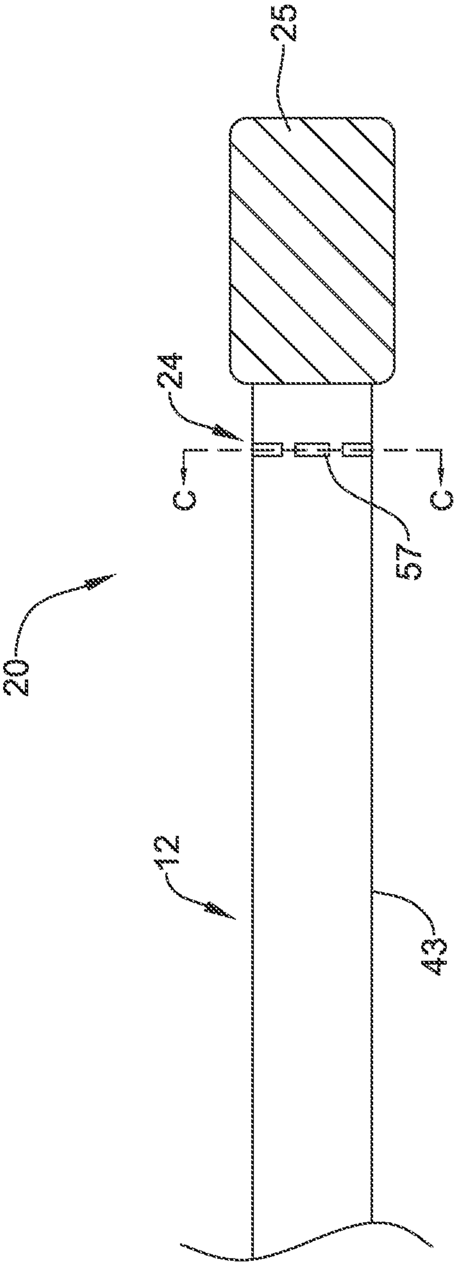


FIG. 9A

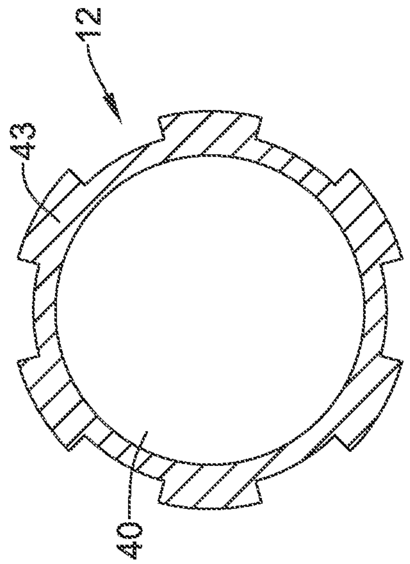


FIG. 9B

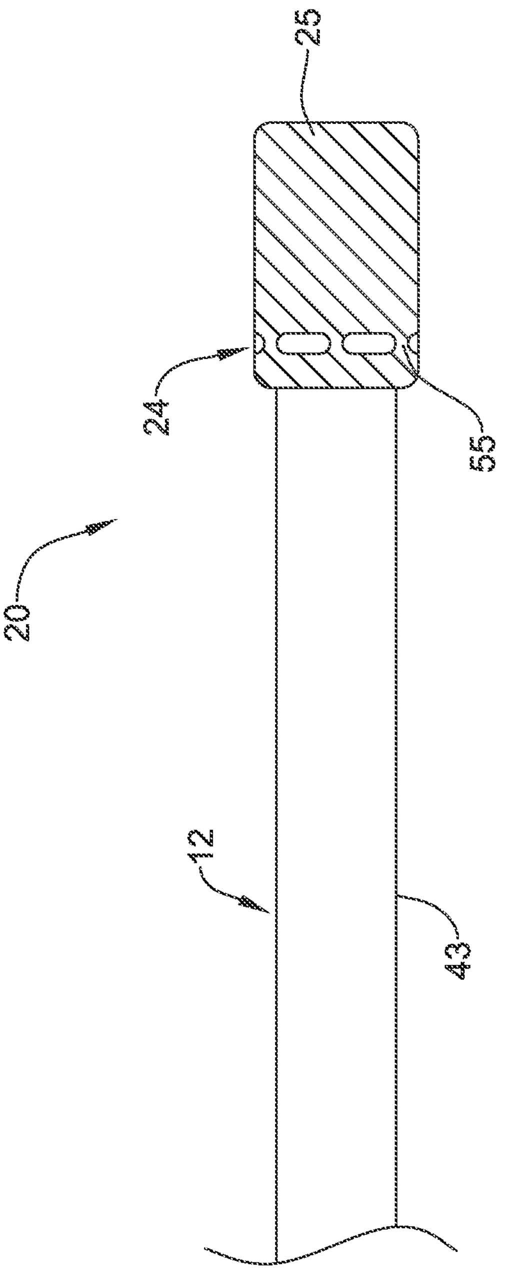


FIG. 10A

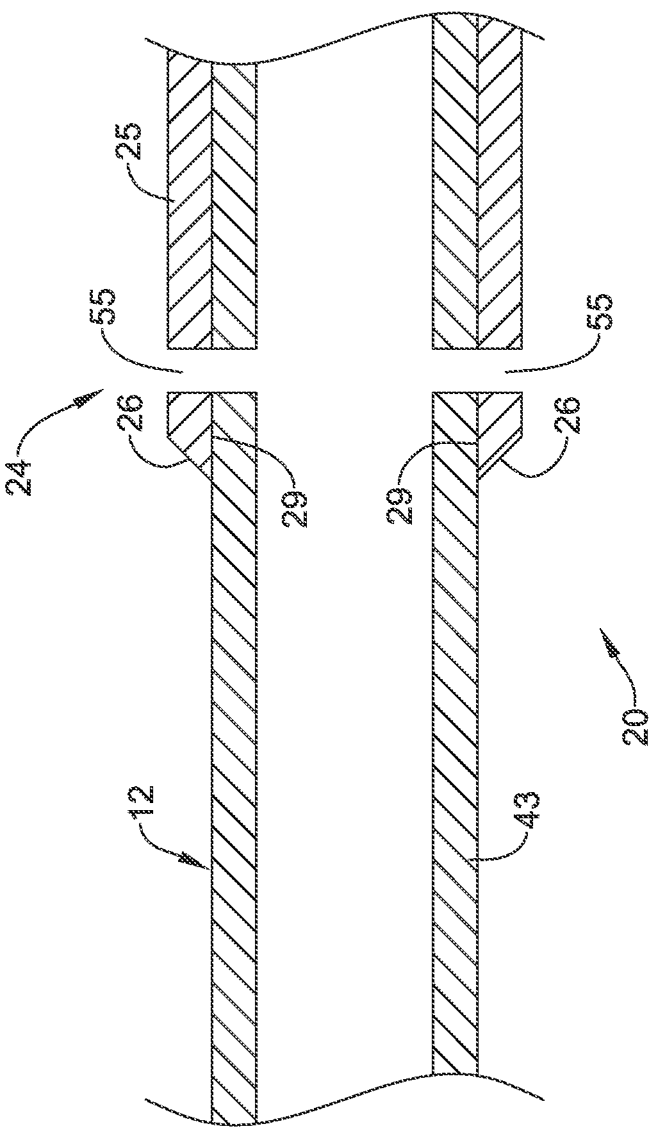


FIG. 10B

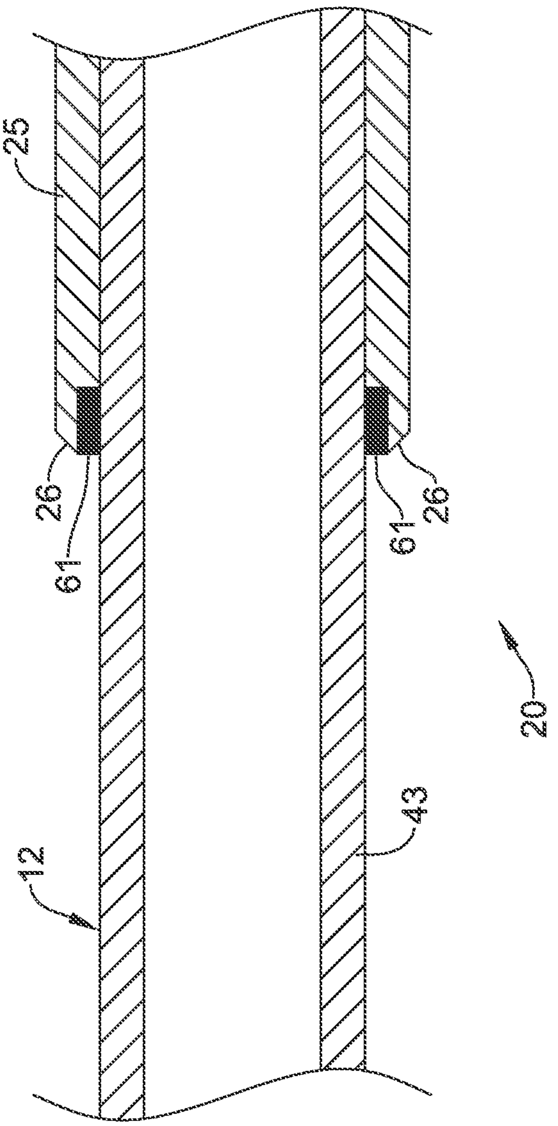


FIG. 11

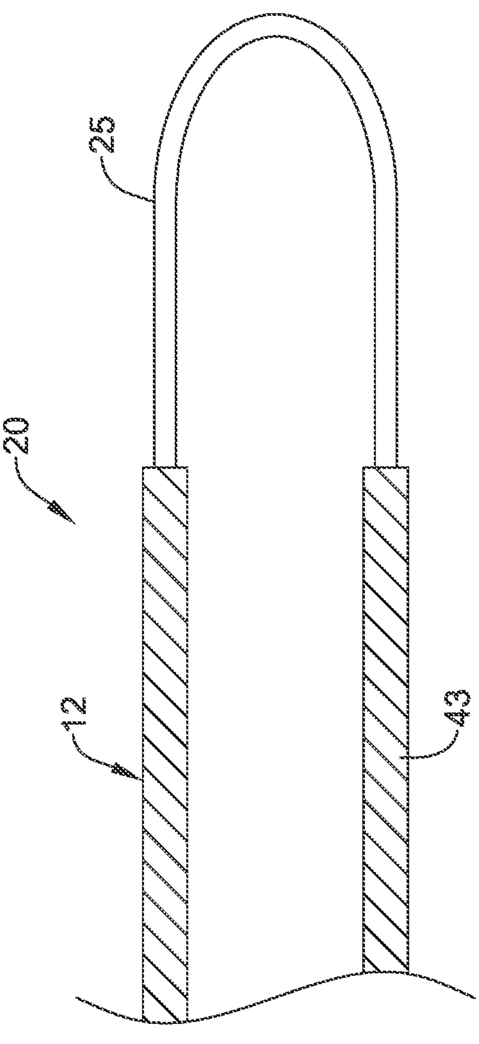


FIG. 12A

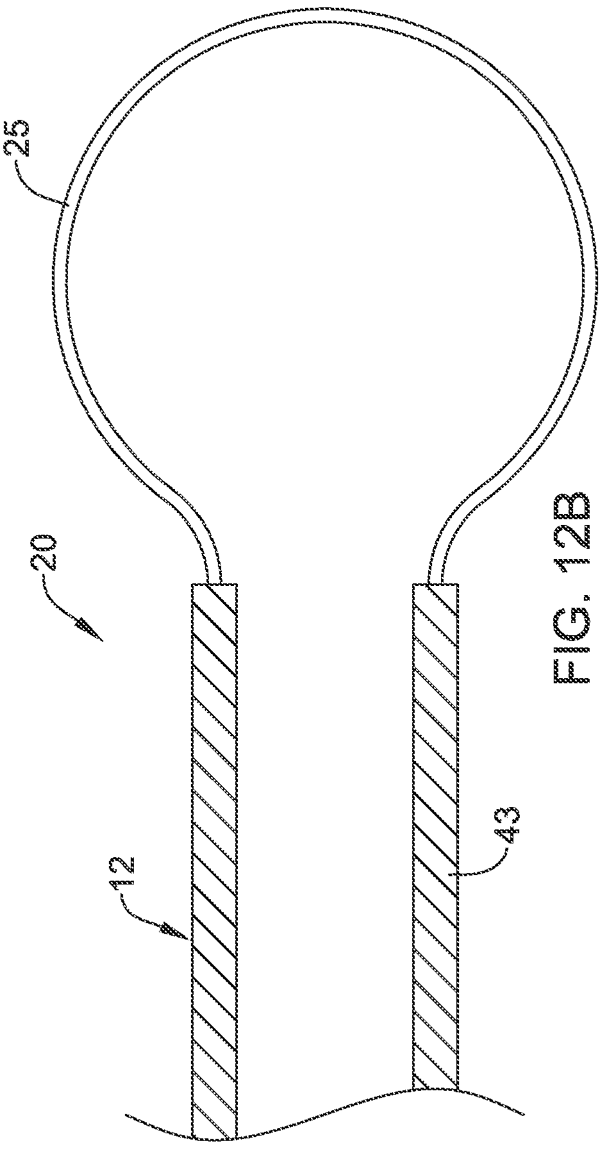


FIG. 12B

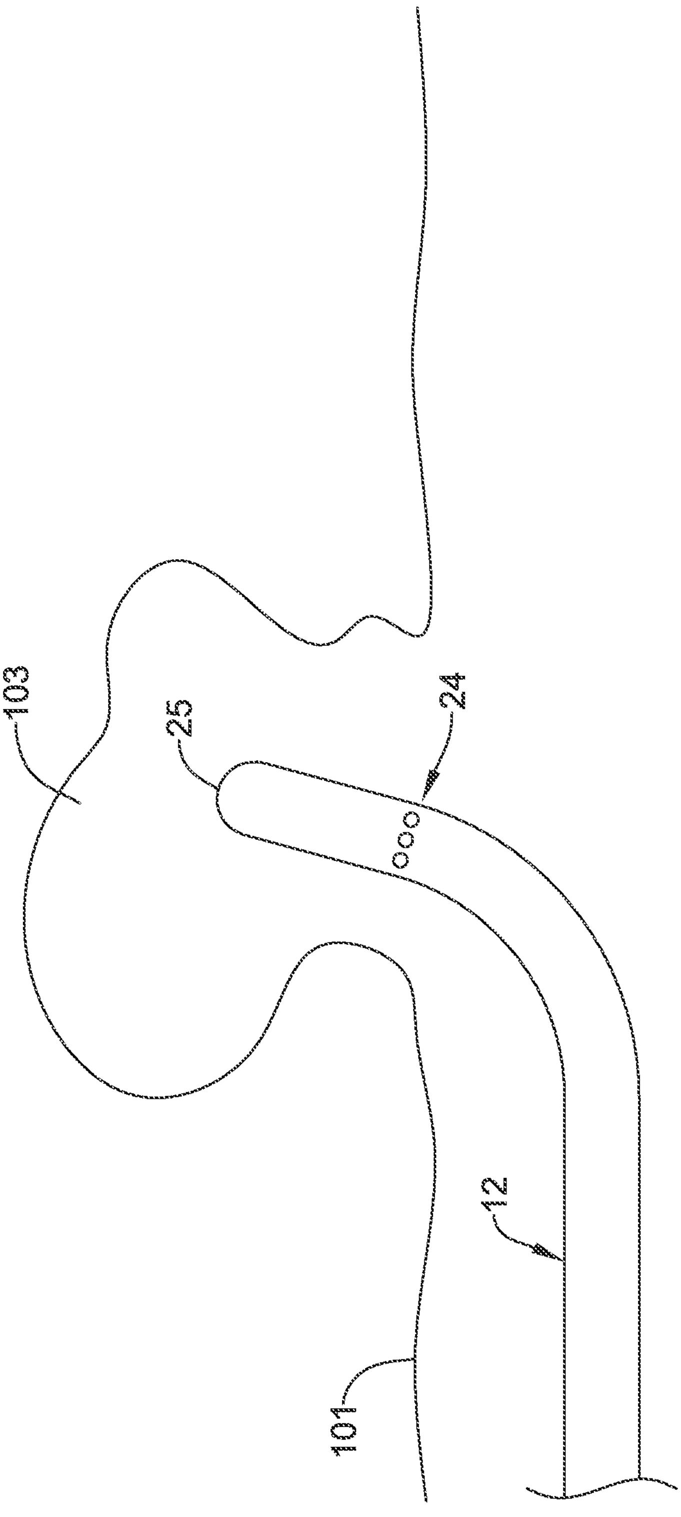


FIG. 13A

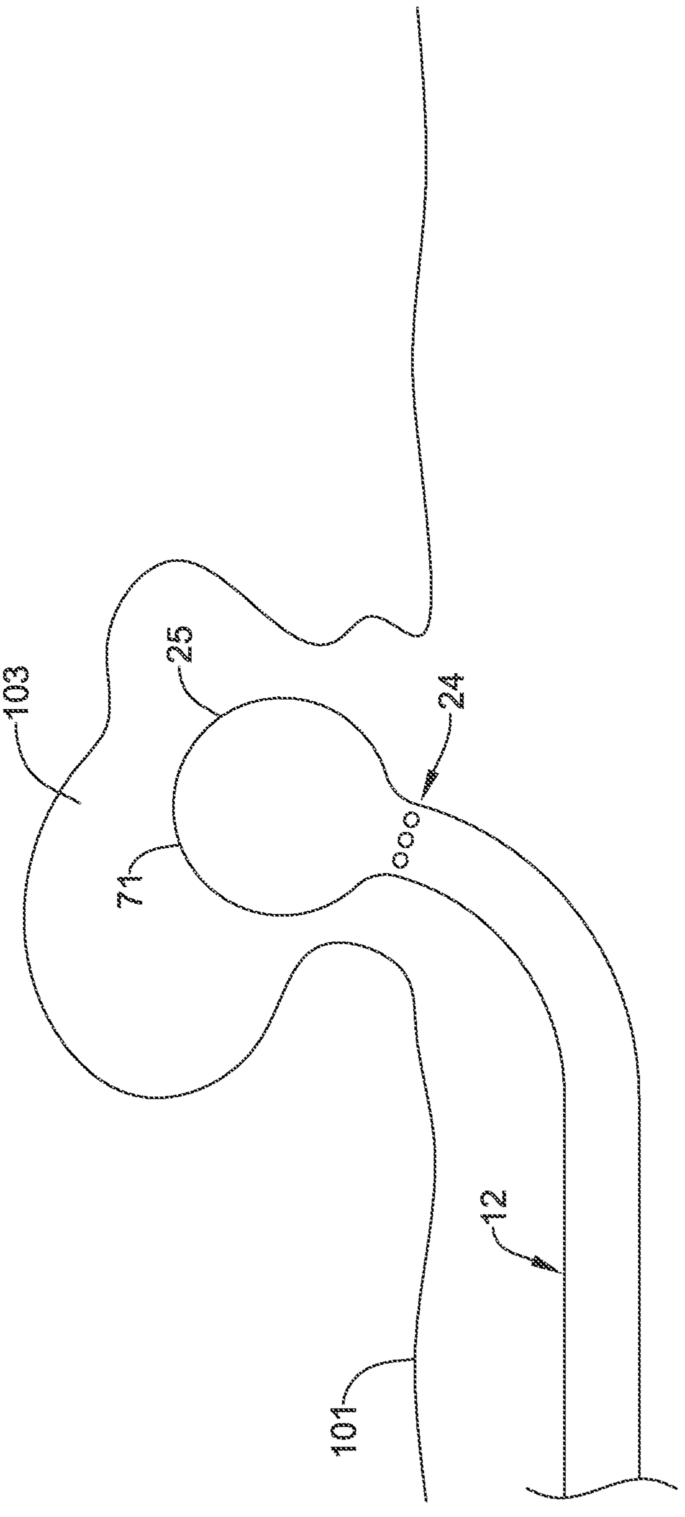


FIG. 13B

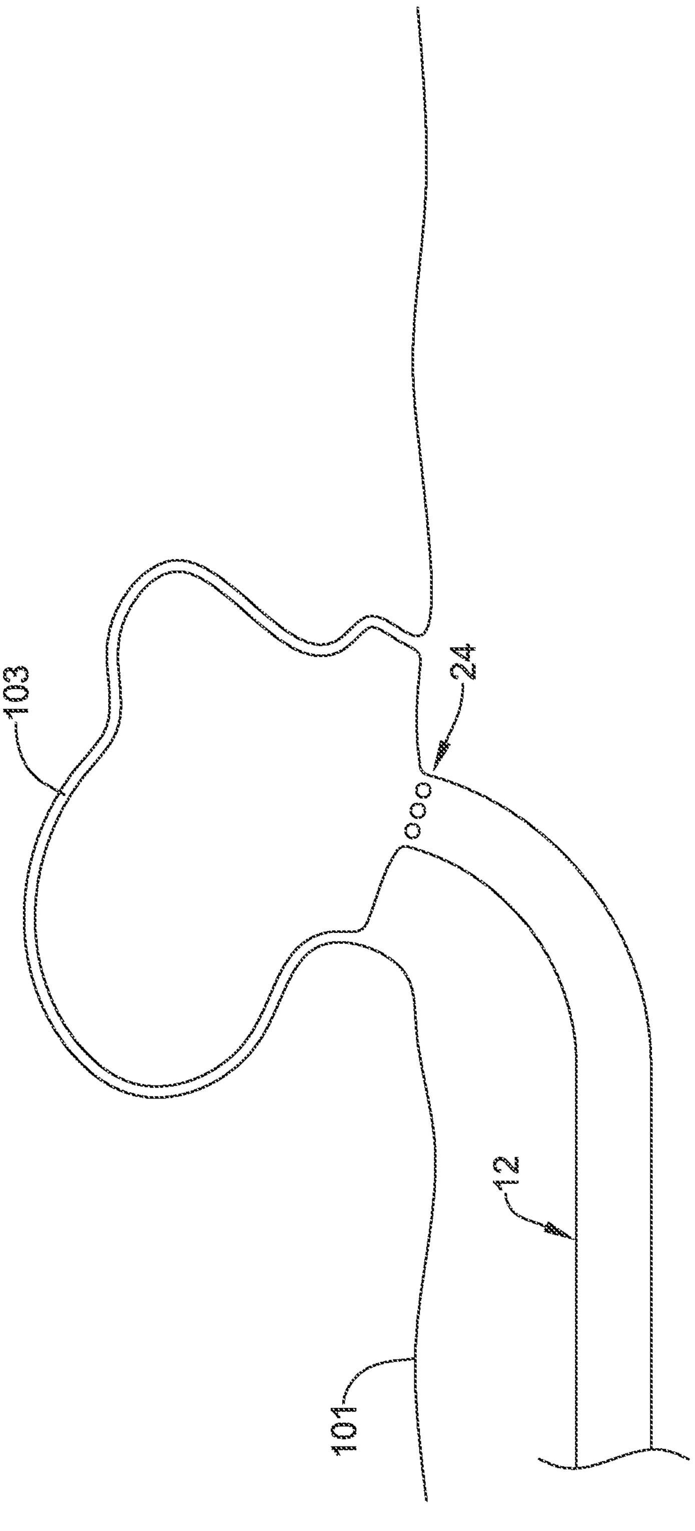


FIG. 13C

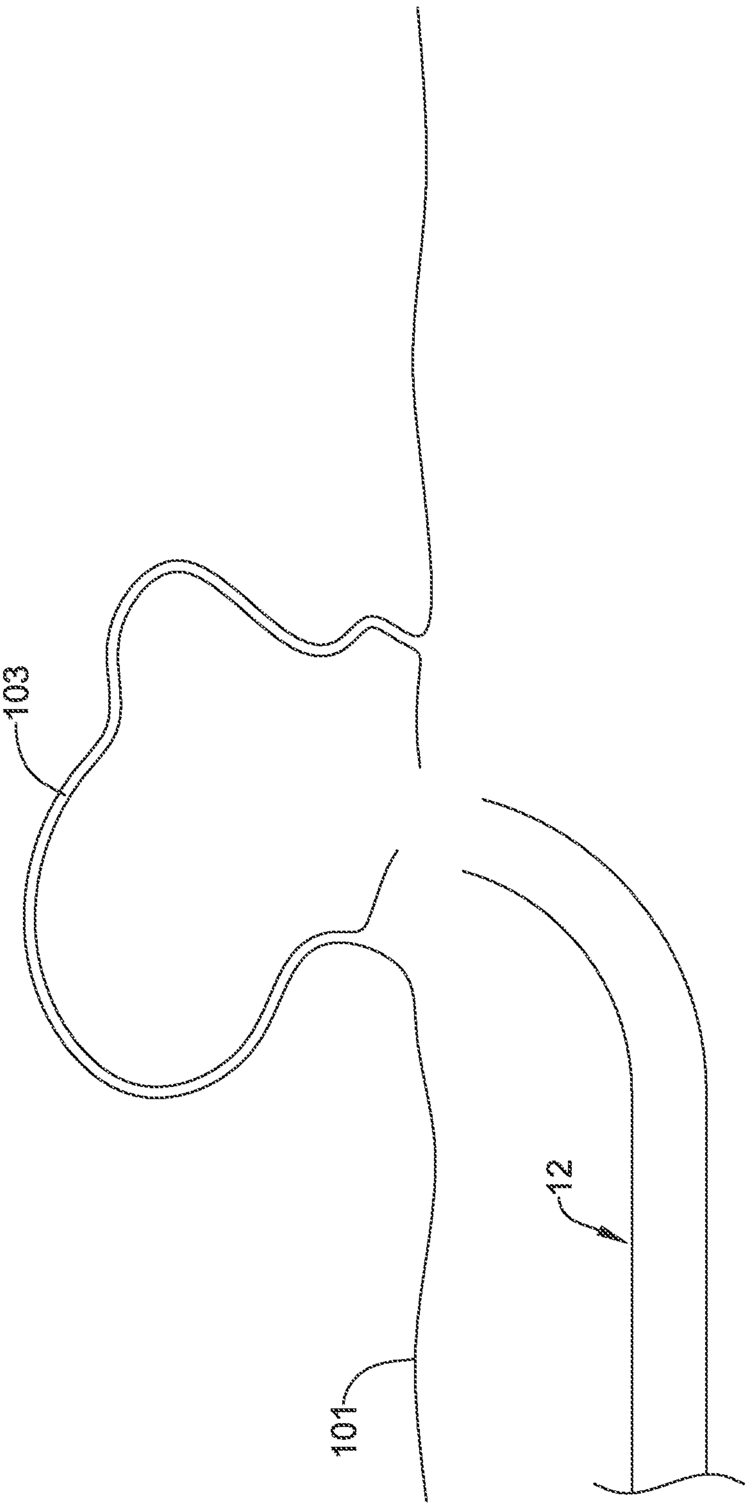


FIG. 13D

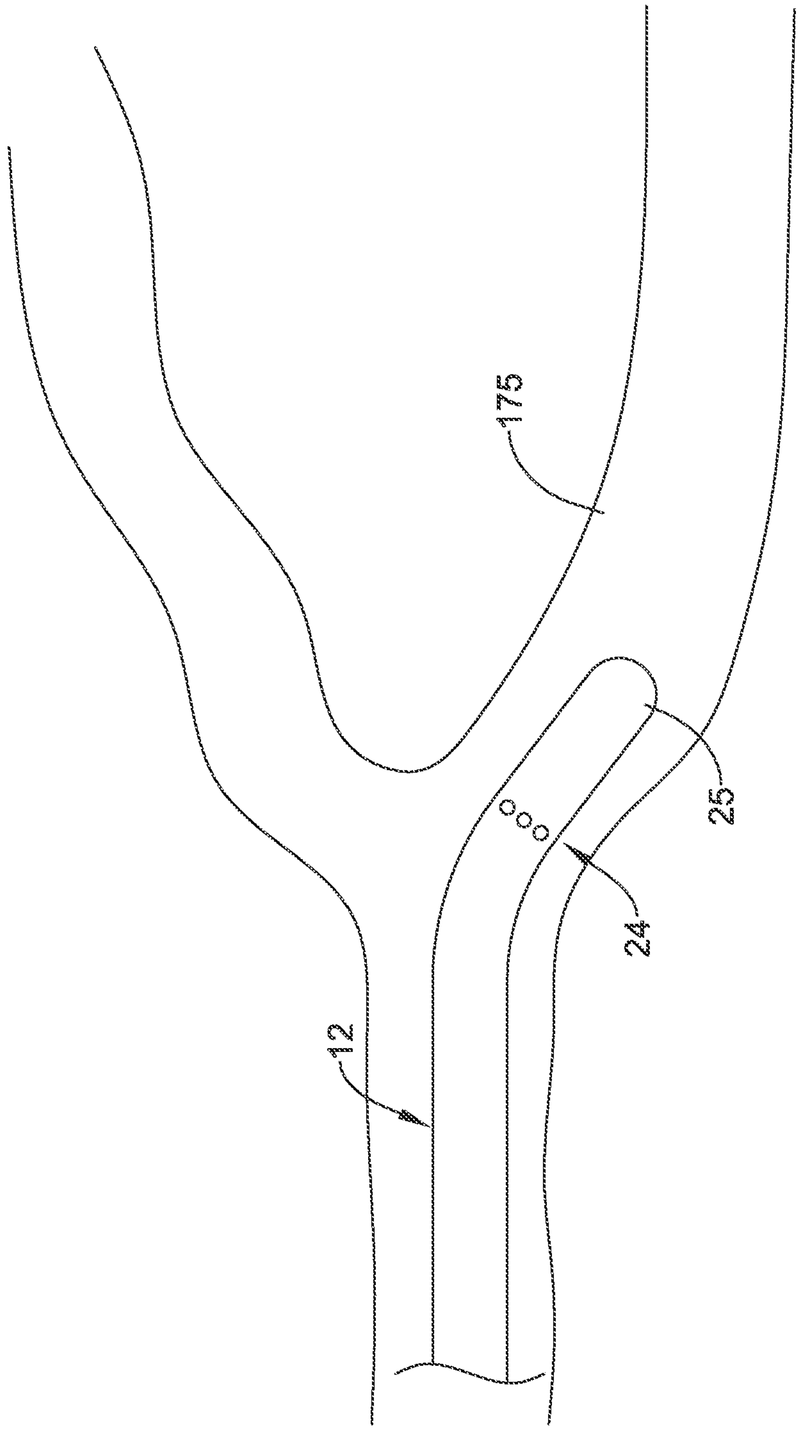


FIG. 14A

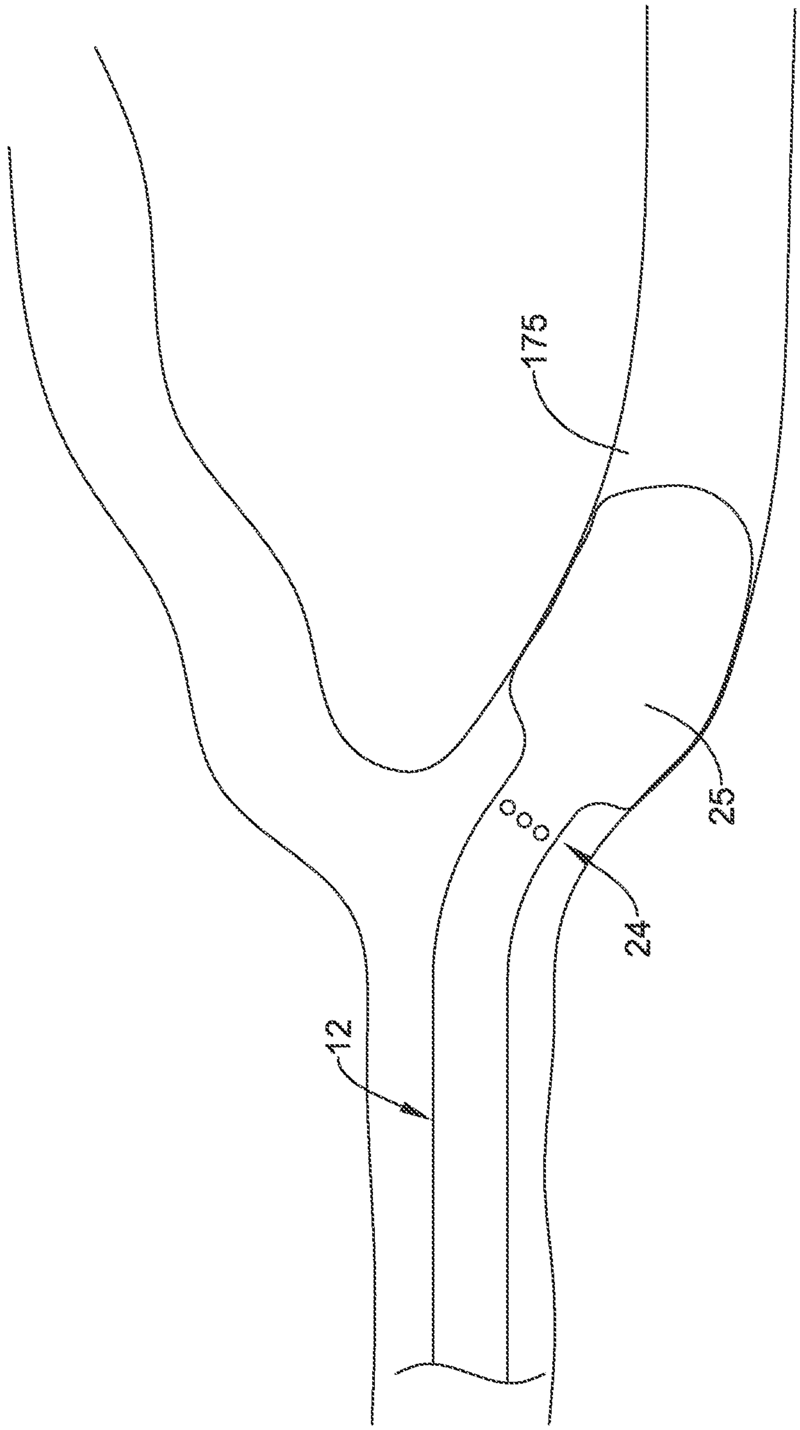


FIG. 14B

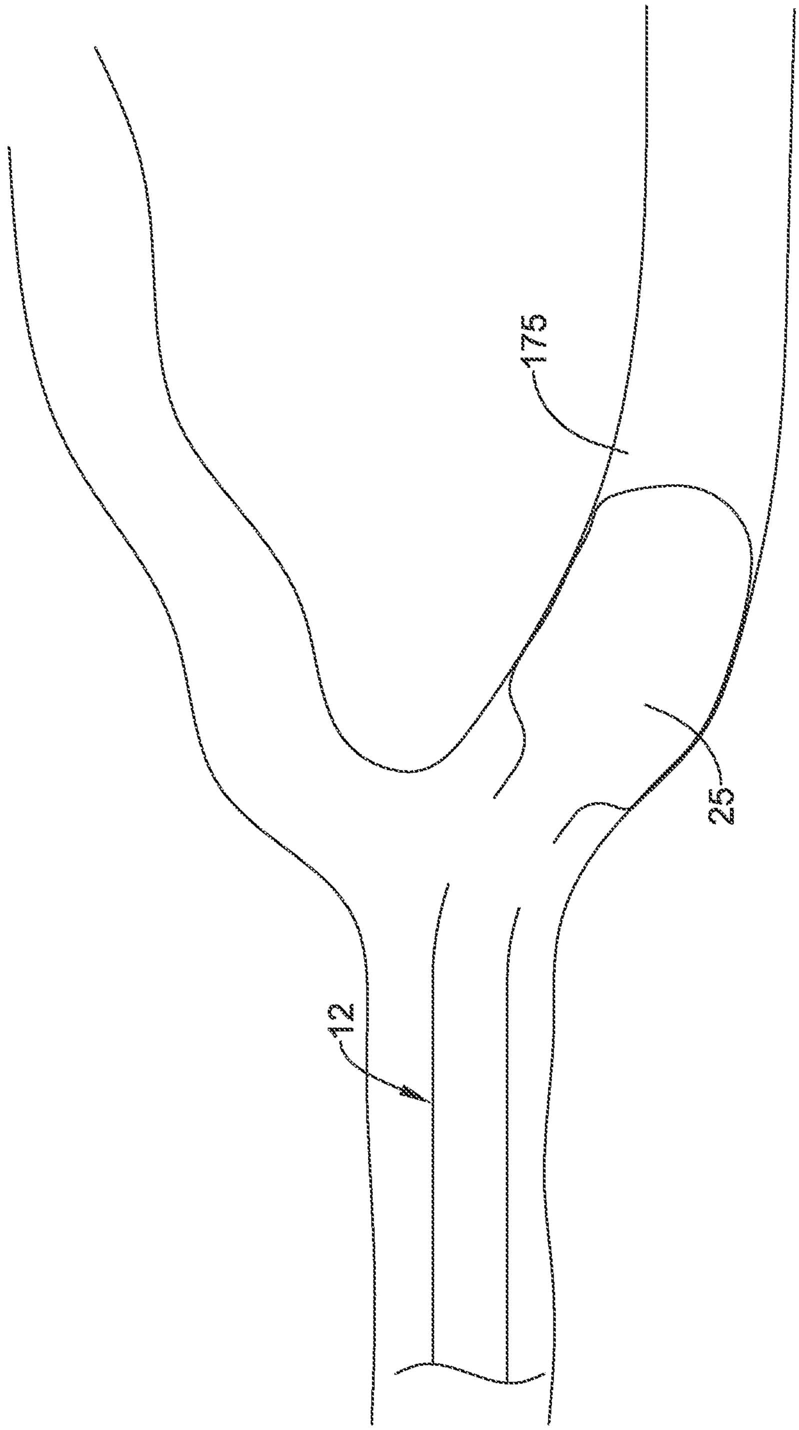


FIG. 14C

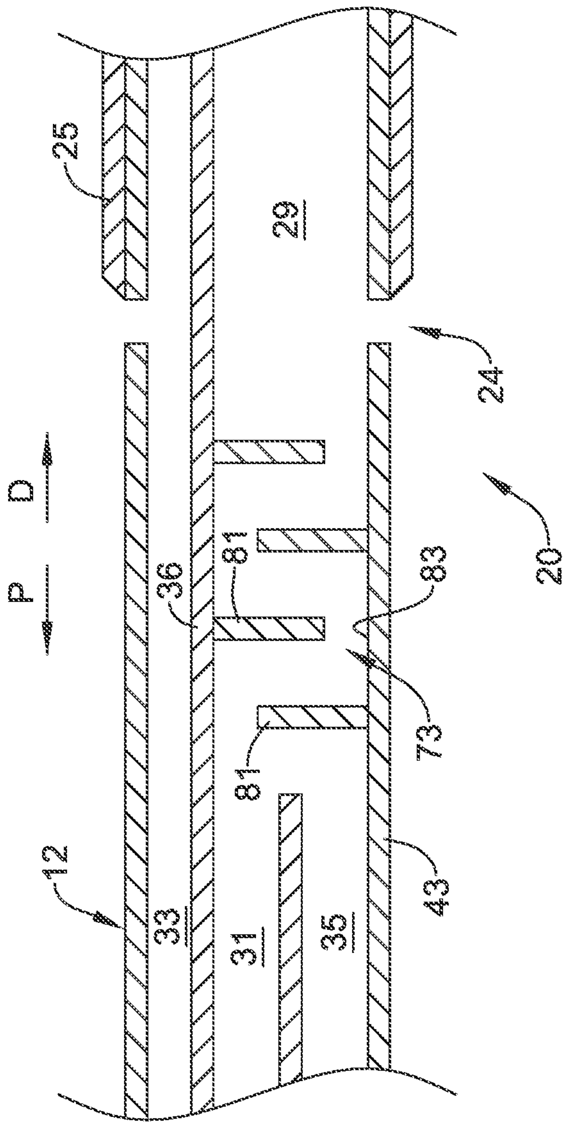


FIG. 15

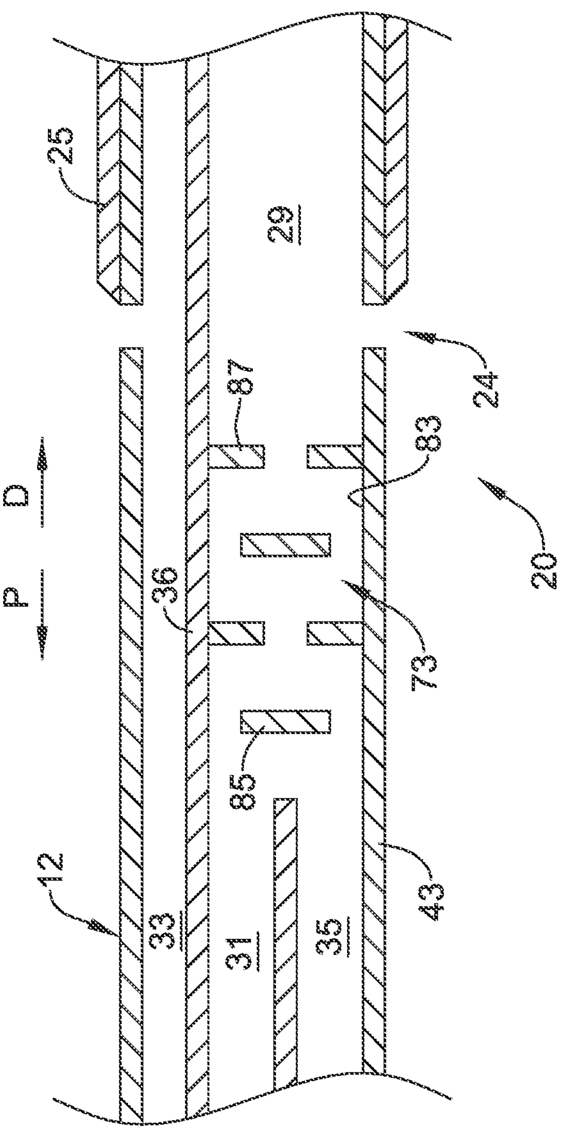


FIG. 16

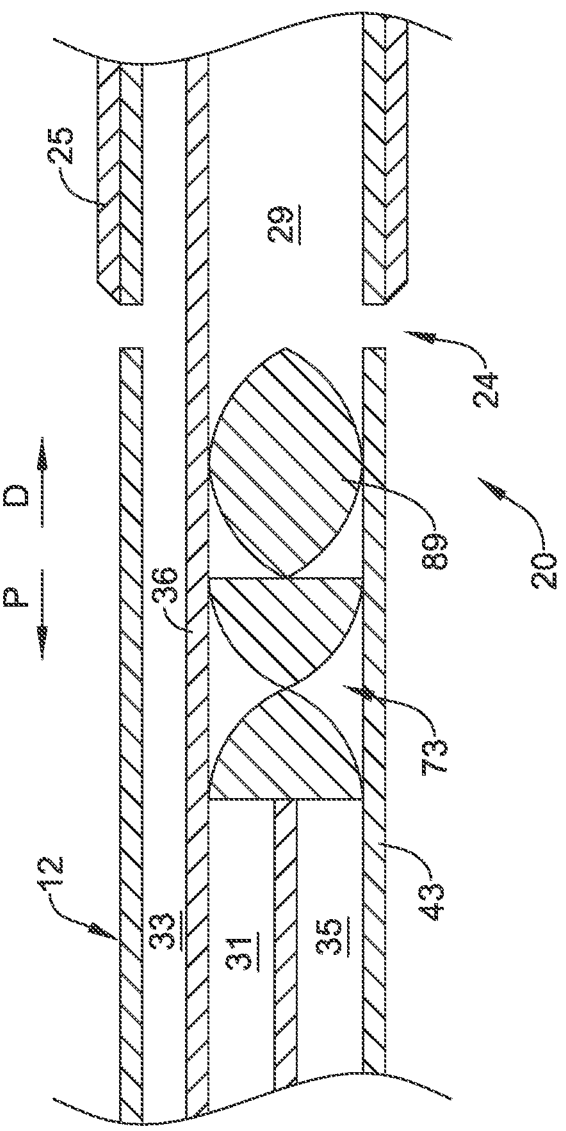


FIG. 17

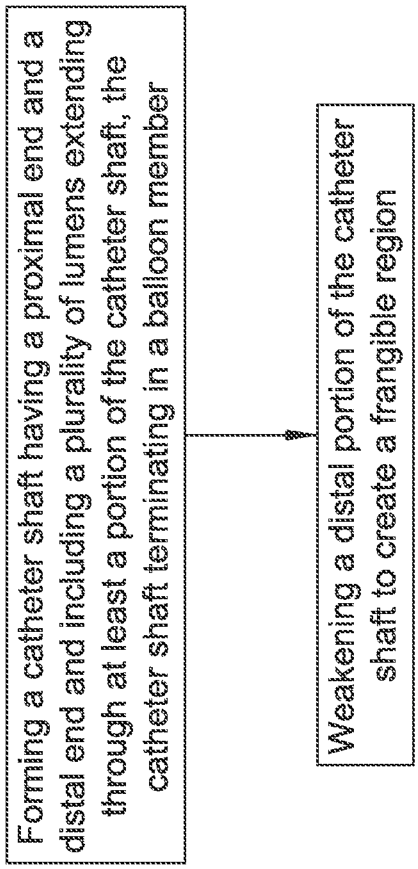
Forming a catheter shaft having a proximal end and a distal end and including a plurality of lumens extending through at least a portion of the catheter shaft, the catheter shaft terminating in a balloon member
Weakening a distal portion of the catheter shaft to create a frangible region

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2003220666 A
- US 8182465 B